# EUROPEAN PATENT SPECIFICATION

(11) **EP 3 914 200 B1**
(45) Date of publication and mention of the grant of the patent: **11.03.2026**
(21) Application number: 20708248.8
(22) Date of filing: 15.01.2020
(51) Int. Cl.: A61F 5/00

(54) **OBESITY TREATMENT DEVICE**
BEHANDLUNGSVORRICHTUNG FÜR ADIPOSITAS
DISPOSITIF DE TRAITEMENT DE L'OBÉSITÉ

(30) Priority: 22.01.2019 US 201962795346 P; 29.07.2019 US 201962879935 P; 04.10.2019 US 201962911009 P
(43) Date of publication of application: 01.12.2021
(73) Proprietor: Lean Medical Technologies, Inc., Ann Arbor, MI 48103 (US)
(72) Inventor: REISIN, Carina, R., Tustin, CA 92782 (US); MURI, John, I., Laguna Niguel, CA 92677 (US); KNOPP, Peter, G., Ann Arbor, MI 48103 (US); LADABAUM, Uri, Ann Arbor, MI 48103 (US); BEEUWSAERT, Michael, San Clemente, CA 92673 (US); GOODMAN, Jack, Ann Arbor, MI 48103 (US); BHAGAT, Vishal, J., Ann Arbor, MI 48103 (US); GUNARATNAM, Naresh, T., Ann Arbor, MI 48103 (US)
(74) Representative: Berggren Oy
(86) International application number: PCT/US2020/013730
(87) International publication number: WO 2020/154149

(56) References cited:
- CN-A- 108 938 163
- US-A1- 2013 030 351
- US-A1- 2016 095 733
- US-B1- 9 622 897

## Description

### FIELD

The present disclosure is related to medical devices. More specifically, the disclosure is related to an obesity treatment device.

### BACKGROUND

Obesity has become an epidemic health crisis in the United States and around the world. (The National Institutes of Health defines obesity as having a body mass index (BMI) of 30 or above.) Currently, nearly three-quarters of American men and more than 60% of American women are obese or overweight. Meanwhile, nearly 30% of boys and girls under age 20 are either obese or overweight, up from 19% in 1980. And over a billion people worldwide are obese. Obesity-related conditions, including heart disease, stroke, Type 2 diabetes and certain types of cancer, are some of the leading causes of preventable death. The estimated annual health care costs of obesity-related illness are $190.2 billion, or nearly 21% of annual medical spending in the United States. The National Institutes of Health estimates that 300,000 deaths in the United States each year are attributed to obesity, the second leading cause of preventable death behind cigarette smoking.

Although diet and exercise programs can be effective in promoting weight loss, it is estimated that less than 5% of people who engage in such programs achieve sustained, long-term weight loss. Surgical weight loss procedures can also be effective, but due to their very invasive nature, they are typically reserved for morbidly obese patients (e.g., a BMI > 40), which is a small percentage of the obese population. These surgical procedures typically involve removal of a portion of the stomach and/or small intestine and/or a reattachment of the stomach to a different portion of the small intestine. The surgical procedures often make post-operative eating and nutrition incredibly challenging, and they are associated with morbidity and mortality rates of about 0.1-2%. The costs of surgical weight loss procedures and post-operative care are also daunting. For example, the commonly performed Roux-en-Y gastric bypass procedure typically costs over $35,000 and includes up to three days of post-surgical hospitalization. Even after such invasive surgical procedures and sometimes radical initial weight loss, many patients who undergo these procedures still regain the weight over time.

In an attempt to provide a less invasive, cost effective treatment for obesity, many different obesity treatment medical devices have been developed over time. For example, expandable balloon-like implants have been placed in the stomach to occupy space and make the patient feel full. Different types of bands have been placed around the outside of the stomach to squeeze the stomach, again to make patients feel full. Similarly, one medical device company developed a balloon for placement outside the stomach, in the abdominal cavity, to press against the stomach from the outside.

Another type of device is a duodenal sleeve, which is implanted via multiple hooks in the first part of the duodenum and extends down the small intestine to prevent absorption of food in the sleeved portion. Unfortunately, these sleeve devices have been plagued with a number of complications, such as device migration (where the device dislodges and moves down the digestive tract), device obstructions, abdominal pain, bleeding, ulceration, perforation, and abscesses. Another company has developed a device that sucks food out of a patient's stomach through a tube sticking out of a hole in the abdomen, after the patient eats a meal. Unfortunately, all of these and other medical device attempts to treat obesity have either been completely ineffective, less effective than traditional obesity surgery, so invasive as to be intolerable for most patients, intolerable due to side effects, or some combination thereof.

Therefore, it would be highly desirable to have a less invasive device and method for treating obesity. Ideally, such a device and method would be relatively simple to deploy and would not require extensive post-operative care or long, costly hospital stays. At the same time, the obesity treatment would ideally be at least nearly as effective as traditional obesity surgery, if not as effective or more effective. It would also be desirable to have a device that could be used on obese patients earlier in the stages of obesity, so that it was not limited to use only in morbidly obese patients. At least some of these objectives will be addressed in the present disclosure.

Document US20130030351 presents a system for delivery and implantation of a gastrointestinal device comprising a flexible thin-walled sleeve and an expandable anchor attached to the proximal end of the sleeve. The device can be inserted endoscopically through the mouth, throat, stomach and intestines.

US 9,622,897 discloses implant systems having components implantable and removable using endoscopic techniques for treatment of obesity, diabetes, reflux, gastroparesis and other gastrointestinal conditions.

### SUMMARY

The pylorus (or "pyloric sphincter") is a constriction in the gastrointestinal tract between the distal end of the stomach and the proximal end of the small intestine (i.e., the duodenum). The main functions of the pylorus are to prevent intestinal contents from reentering the stomach when the small intestine contracts and to limit the passage of large food particles or undigested material into the intestine. Studies have shown that when passage of food through the pylorus from the stomach into the duodenum is slowed, for example in patients with a natural obstruction of the pylorus, patients tend to lose weight.

Thus, in general, the obesity treatment device described in this disclosure includes a pyloric implant, which is placed across the pylorus, from the distal end of the stomach to the proximal end of the duodenum, and which is designed to slow passage of food through the pylorus. The pyloric implant may also be referred to by other names, such as a "stent," "valve," "controller," "blocker" or the like. It typically includes a stomach portion (or "stomach anchoring portion" or "proximal portion") at one end, a pyloric portion (or "pylorus spanning portion"), and a duodenal portion (or "duodenal anchoring portion" or "distal portion") at an opposite end. When delivered into the digestive tract from a delivery device (such as a catheter), the stomach portion and the duodenal portion expand to larger maximum diameters than the pylorus spanning portion, thus giving many embodiments of the device an overall shape similar to that of an hourglass.

Embodiments of the obesity treatment device described in this application include at least one support member (or "frame"), which forms the shape of the device, and a material (or "implant material") disposed over the support member. Many different embodiments and features of these two components are described below. Embodiments of the obesity device also include a restrictor, which is coupled with the frame of the device to restrict passage of food through the pyloric portion. In other embodiments, the shape of the device is designed to restrict passage of food, eliminating the need for a separate restrictor piece. In many embodiments, the pyloric implant is self-expanding and is delivered through a flexible, tubular delivery device passed into the stomach. The prosthesis thus often includes one or more shape memory materials that expand upon delivery of the device across the pylorus. Once expanded in position in the patient, the shape and framework of the obesity device help anchor it in place and prevent the device from passing out of the pylorus and into the small intestine or the stomach.

The present invention relates to a device as set out in the claims.

In some embodiments, the first maximum diameter is larger than the third maximum diameter. In some embodiments, an inner diameter of the channel increases between a proximal opening of the pyloric portion at the stomach portion and a distal opening of the pyloric portion at the duodenal portion. In alternative embodiments, an inner diameter of the channel decreases between a proximal opening of the pyloric portion at the stomach portion and a distal opening of the pyloric portion at the duodenal portion.

In some embodiments, the support member of the stomach portion, the pyloric portion and the duodenal portion is a single shape memory support stent that extends from a proximal end of the stomach portion, through the pyloric portion, to a distal end of the duodenal portion. Alternatively, the support member maybe be three (or some other number of) attached pieces of shape memory stent material, one for each of the stomach portion, the pyloric portion and the duodenal portion. In some embodiments, the support member of the stomach portion and the support member of the duodenal portion are each made of a shape memory material, and the support member of the pyloric portion is a different material attached at each end to the support member of the stomach portion and the support member of the duodenal portion. For example, the different material might be a compliant material, and the shape memory material might be Nitinol or any other shape memory metal.

In some embodiments, the implant material is two layers of material, and the support member is disposed between the two layers. Some embodiments may also include a coating applied to an inner surface and/or an outer surface of the device. For example, some embodiments may include a bioadhesive coating on the outer surface of the device, to help hold the device in place after delivery. In some embodiments, the implant material itself may be a coating on one or both sides of the support member. The support member may be one-piece or multiple pieces and may have any suitable shape(s) and size(s), such as but not limited to rings, star shaped members, a continuous wire extending from the stomach portion to the duodenal portion, and braided wire.

In some embodiments, the feature of the device configured to slow the passage of the food material through the channel is an inner diameter of at least part of the pyloric portion of the device. In some embodiments, the device further includes a restrictor attached to the support member at or near the pyloric portion. In such embodiments, the feature of the device configured to slow the passage of the food material through the channel is the restrictor. In some embodiments, the restrictor is located at a junction between the stomach portion and the pyloric portion. Other embodiments may include a restrictor built into the support member at or near the pyloric portion.

Any of the embodiments described in this application may also include a duodenal sleeve attached to a distal end of the duodenal portion, for reducing or eliminating absorption of nutrients by the duodenum. In some embodiments, the duodenal sleeve may include one or more restrictors coupled with or formed in the duodenal sleeve to slow passage of food through the duodenal sleeve. In yet another embodiment, the pyloric portion may include a shape memory material configured to change shape and expand when a predefined amount of pressure is applied to the stomach portion by food in the stomach. For example, the pyloric portion might be configured to change from a default, twisted configuration to an open, untwisted configuration, in response to the predefined amount of pressure applied to the stomach portion by food in the stomach. The predefined amount of pressure may include a range of pressures from a smallest amount of pressure at which the pyloric portion starts to untwist to a largest amount of pressure, which is required for the pyloric portion to completely untwist. As a non-limiting example, the smallest amount of pressure might be between 0.0197 bar (20 mmHg) and 0.0395 bar (40 mmHg), and the largest amount of pressure might be at least 0.0790 bar (80 mmHg).

### BRIEF DESCRIPTION OF THE DRAWINGS

Fig. 1 is a cross-sectional view of a distal end of a stomach and a proximal end of the duodenum, with a weight loss implant device in position in the pylorus and an optional sleeve component extending into the duodenum, according to one embodiment described in the Incorporated References;
Fig. 2 is a cross-sectional view of the pyloric implant portion of the device of Fig. 1, also as described in the Incorporated References;
Figs. 3A and 3B are side and perspective views, respectively, of a pyloric implant, according to one embodiment;
Figs. 4A and 4B are perspective and cross-sectional/perspective views, respectively, of a pyloric implant, according to another embodiment;
Figs. 5A and 5B are perspective and cross-sectional/perspective views, respectively, of a pyloric implant, according to another embodiment;
Fig. 6 is a side view of a pyloric implant, illustrating relative sizes of the parts of the implant, according to one embodiment;
Fig. 7 is a side view of a pyloric implant, illustrating relative sizes of the parts of the implant, according to another embodiment;
Fig. 8 is a side view of a pyloric implant, illustrating relative sizes of the parts of the implant, according to another embodiment;
Figs. 9A-9D are side views of four different general types of embodiments of a pyloric implant;
Figs. 10A-10D are side, rear perspective, cross-sectional and front perspective views, respectively, of a pyloric implant with support rings at opposite ends of the device, according to general embodiment type one;
Fig. 11 is a perspective view of a pyloric implant, according to another embodiment;
Fig. 12 is a perspective view of a pyloric implant, according to another embodiment;
Figs. 13A-13D are three perspective views and a side view of a pyloric implant with braided support members, according to general embodiment type two;
Figs. 14A-14C are perspective views of three different embodiments of braided support members for a pyloric implant, where each implant includes two support members;
Figs. 15A-15C are perspective views of three different embodiments of braided support members for a pyloric implant, where each implant includes one support member;
Figs. 16A-16C are perspective, exploded and side views, respectively, of a pyloric implant with star-shaped support members, according to general embodiment type three;
Fig. 17 is a perspective view of a pyloric implant, according to another embodiment;
Figs. 18A-18C are side, perspective and exploded views, respectively, of a pyloric implant with continuous wire support member, according to general embodiment type four;
Figs. 19A and 19B are perspective views of two different embodiments of pyloric implants with differently configured continuous wire support members;
Figs. 20A and 20B are perspective and side views, respectively, of a pyloric implant with a continuous wire support member, according to another embodiment;
Figs. 21A and 21B are perspective and side views, respectively, of a pyloric implant with multiple wire support members, according to an alternative embodiment;
Fig. 22 is a side view of a pyloric implant with a continuous wire support member, according to another embodiment;
Fig. 23 is a side view of a pyloric implant with surface features, according to one embodiment;
Figs. 24A and 24B are perspective views of a pyloric implant for use with a removable restrictor (not shown), according to one embodiment; and
Fig. 25 is a perspective view of a removable restrictor for use in a pyloric implant, such as the one shown in Figs. 24A and 24B, according to one embodiment;
Fig. 26 is a side, cross-sectional view of a human stomach, pylorus and duodenum, with an obesity treatment device in place across the pylorus;
Figs. 27A and 27B are side and front view of an obesity treatment device with a pressure based restrictor, according to one embodiment;
Figs. 28A and 28B are side and front view of an obesity treatment device with a pressure based restrictor, according to an alternative embodiment;
Figs. 29A and 29B are side and front view of an obesity treatment device with a pressure based restrictor, according to one embodiment;
Figs. 30A-30C are cross-sectional, side and perspective views, respectively, of an obesity treatment device with a pressure-based restrictor, according to one embodiment;
Fig. 30D is a perspective view of the restrictor of the obesity treatment device of Figs. 30A-30C;
Figs. 31A-31C are cross-sectional, side and perspective views, respectively, of an obesity treatment device with a pressure-based restrictor, according to one embodiment;
Figs. 32A and 32B are perspective and side cross-sectional views, respectively, of an obesity treatment device including a pyloric spanning portion and a duodenal sleeve, according to one embodiment; and
Fig. 33 is a side, exploded view of an obesity treatment device including a pyloric spanning portion and a duodenal sleeve, according to one embodiment;
Fig. 34 is a side, exploded view of an obesity treatment device including a pyloric spanning portion and a duodenal sleeve, according to one embodiment;
Figs. 35A and 35B are side and perspective views, respectively, of an obesity treatment device with differently shaped proximal and distal portions, according to one embodiment;
Fig. 35C is a side view of a braided version of the obesity treatment device of Figs. 35A and 35B, according to one embodiment;
Figs. 36A-36C are side perspective, front perspective and rear perspective views, respectively, of an alternative embodiment of an obesity treatment device with a central restrictor;
Fig. 36D is a perspective view of a wire support member and central restrictor of the device of Figs. 36A-36C;
Fig. 36E is a perspective view of the wire support member and central restrictor of Fig. 36D, with optional additional support members;
Figs. 37A and 37B are side perspective (partially transparent) and front perspective views, respectively, of another alternative embodiment of an obesity treatment device;
Fig. 37C is a perspective view of a support member and central restrictor of the device of Figs. 37A and 37B;
Figs. 38A-38C are side, perspective and cross-sectional views, respectively, of an obesity treatment device with two toroidal support members, according to another alternative embodiment;
Figs. 39A-39C are side, perspective and cross-sectional views, respectively, of an obesity treatment device with a toroidal support member and an attached duodenal sleeve, according to another alternative embodiment;
Figs. 40A and 40B are side and perspective views, respectively, of an obesity treatment device with an anchoring portion and a duodenal sleeve, where the restrictor is formed as a narrowed portion of the sleeve, according to another alternative embodiment;
Figs. 41A and 41B are partially transparent side and perspective views, respectively, of an obesity treatment device with an anchoring portion and a duodenal sleeve, where the restrictor is formed as an insert in the sleeve, according to another alternative embodiment;
Figs. 42A and 42B are side and perspective views, respectively, of an obesity treatment device with an anchoring portion and a duodenal sleeve, where the restrictor is an attachment on a distal end of the sleeve, according to another alternative embodiment;
Fig. 43 is a side view of an obesity treatment device with a sleeve that includes multiple, cone-shaped restrictor inserts, according to one embodiment;
Fig. 44 is a side view of an obesity treatment device with a sleeve that includes multiple constricted portions that act as restrictors, according to one embodiment;
Figs. 45A-45C are side views of an obesity treatment device with a twisting closure pyloric portion, in a default, twisted configuration (Fig. 45A), a partially untwisted configuration (Fig. 45B), and a completely untwisted configuration (Fig. 45C), according to one embodiment;
Figs. 46A-46C are front views of the obesity treatment device of Figs. 45A and 45B, in the twisted configuration (Fig. 46A), the partially untwisted configuration (Fig. 46B), and the completely untwisted configuration (Fig. 46C).

### DETAILED DESCRIPTION

The disclosed obesity treatment device addresses one or more of the disadvantages of traditional obesity surgical procedures and previously developed obesity devices, by providing a safe, easily removable device with improved weight loss performance. As mentioned above, the present disclosure describes a pyloric implant for slowing gastric emptying to prolong and/or increase satiety. The present disclosure focuses primarily on obesity treatment devices (or portions thereof) that span the pylorus, from an anchoring portion in the stomach to an anchoring portion in the duodenum. The disclosure also describes multiple embodiments of an optional duodenal sleeve, which may be attached to the duodenal anchoring portion of the pyloric implant. More detailed descriptions of duodenal sleeves, connection of such sleeves to the pyloric spanning portions of the device, delivery/insertion tools and methods and the like may be found in the Incorporated References. Any of the pyloric implant embodiments described herein may be used with (or adapted for use with) any of the sleeves or delivery devices described in the Incorporated References. They may also be used (or adapted for use with) any other suitable duodenal sleeves and/or delivery devices that are not described in the Incorporated References, and any features described in this or other applications may be combined with features of other embodiments to provide different embodiments.

In various embodiments, the pyloric implant described herein may be used to treat obesity and may also be used to treat Type 2 diabetes in obese and non-obese patients. The device may be easily and safely deployed, either endoscopically or radiologically. When a desired amount of weight loss is achieved, the device may be easily and atraumatically removed.

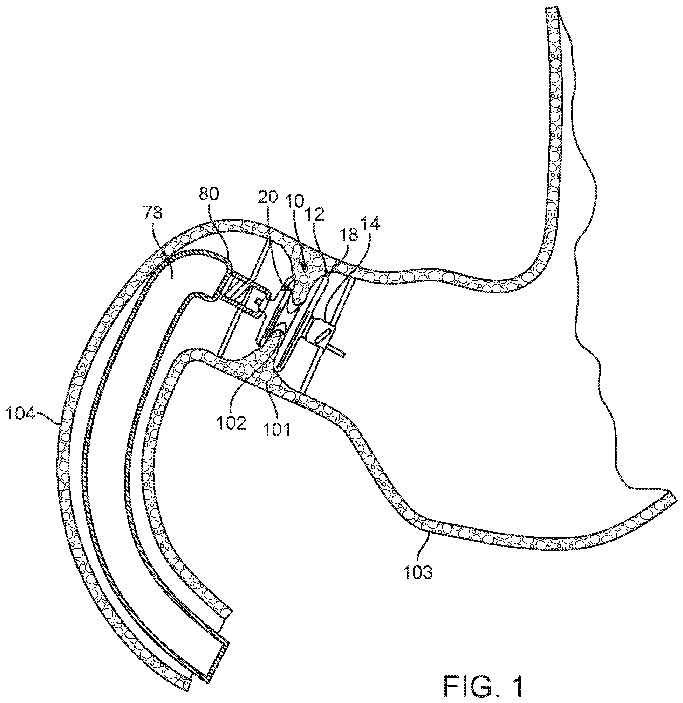

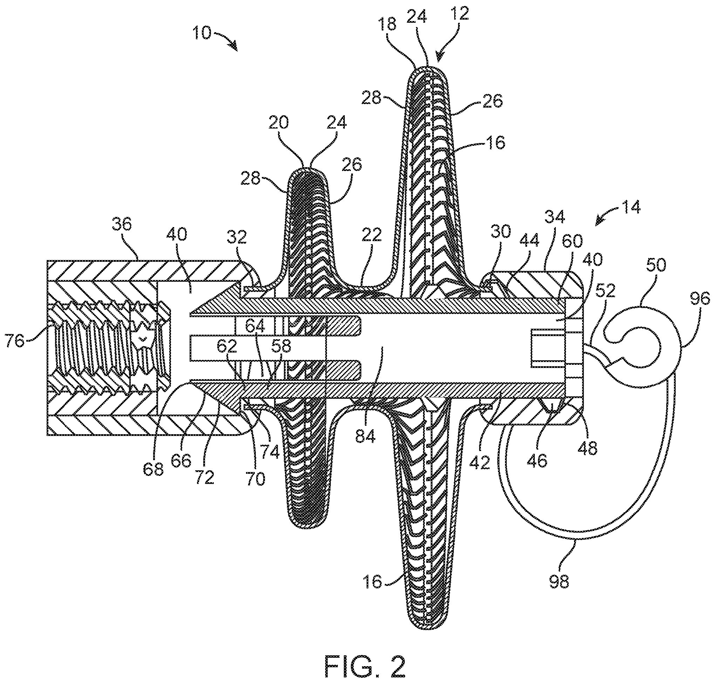

Referring now to Fig. 1, one embodiment of a pyloric implant 10 is shown in its implanted position, extending from a stomach 103, through the pylorus 101 and into the duodenum 104. The prosthesis 10 is shown here attached to a sleeve 78, but as mentioned above, the sleeve 78 is optional. Fig. 2 illustrates the same embodiment of the prosthesis 10 in greater detail in cross section. The pyloric implant 10 is described in this application as a gastrointestinal device designed for a specific use in the pylorus. In alternative embodiments, however, the pyloric implant 10 may be used (or adapted for use) for any application in which a reduction in flow is desired between two regions of the body. Although these alternative embodiments are not described herein, any embodiments in this application may be used or adapted for use in any other suitable part of the body.

Generally, the pyloric implant 10 and other embodiments described herein have one end that resides in the stomach and an opposite end that resides in the duodenum, once the device is inserted across the pylorus. The end in the stomach may sometimes be referred to as the "proximal end" or the "front end" of the device, while the end in the duodenum may be referred to as the "distal end" or the "back end" of the device. Typically, the pyloric implant 10 (and alternative embodiments) will include a restrictor of some kind, to slow the passage of food through the pylorus 101. In some embodiments, the restrictor may simply be a shape or built-in feature of the pyloric implant 10. In alternative embodiments, the restrictor may be a separate piece (or pieces) that are attached to the pyloric implant 10. In either case, the restrictor (or shape/feature) may be located anywhere along the length of the pyloric implant 10, in other words at the distal end, the proximal end, or anywhere in between. Although there may be some advantages in positioning the restrictor closer to the stomach-facing proximal end of the device, this is not required and may not be the case in some embodiments. Furthermore, any embodiment of the restrictors described herein may be used in any embodiment of the pyloric implant 10 described herein. Although every possible permutation of features will not be described herein, the scope of the present disclosure is meant to extend to all feasible variations and combinations.

As shown in Fig. 1, the pyloric implant 10 may be configured to straddle the pylorus 101 or pyloric sphincter 102, which connects the stomach 103 to the duodenum 104. The pyloric implant 10 may include a main body 12 and a connector assembly 14. The main body 12 may include a plurality of strands or wires 16 (Fig. 2). The strands 16 may be formed of any suitable material, such as a metal or polymer. In at least one embodiment, the strands 16 are formed of a shape-memory or heat-formable material. The strands 16 may also be formed of a highly elastic material, for example, a material that exhibits superelasticity, such as but not limited to Nitinol. The strands 16 may be woven or braided together, or they may be un-woven, separate strands 16.

In general, the strands 16 of the main body 12 may be formed into a stomach portion 18 (or "proximal" or "stomach anchoring" portion) and a duodenal portion 20 (or "distal" or "duodenal anchoring" portion), with a pylorus spanning portion 22 between the two. The stomach portion 18 and the duodenal portion 20 each have a larger diameter than the pylorus spanning portion 22, such that the larger diameters are configured to be larger than a maximum diameter of the pylorus, to prevent distal or proximal movement of the main body 12.

The stomach portion 18 and the duodenal portion 20 may be disc or pancake shaped, such that they taper from the diameter of the pylorus spanning portion 22 to a maximum diameter 24 and back to a reduced diameter. The stomach portion 18 and the duodenal portion 20 may each define proximal and distal opposing surfaces 26 and 28. For example, the stomach portion 18 may form a proximal surface 26 or face and an opposing distal surface 28 or face, and the duodenal portion 20 may form the same. Accordingly, the proximal surface 26 of the stomach portion 18 may face the stomach 103, and the distal surface 28 of the stomach portion 18 may face the antral side of the pylorus 101. The proximal surface 26 of the duodenal portion 20 may face the distal side of the pylorus 101, and the distal surface 28 of the duodenal portion 20 may face the duodenal bulb.

The proximal portion 18 of the main body 12 may have a larger diameter (e.g., maximum diameter) than the distal portion 20. Since partially digested food (e.g., chyme) flows from the stomach into the small intestine, there will be a greater force or pressure on the pyloric implant 10 in the proximal to distal direction. Therefore, the stomach portion 18 may have a larger diameter, in order to more effectively resist the pressure from the flow of partially digested food. Reducing the diameter of the duodenal portion 20 relative to the stomach portion 18 may reduce the area of interaction between the device 10 and the duodenum 104. This reduced diameter will reduce the risk for irritation of the duodenal tissue lining and avoid adverse effects, such as ulceration and bleeding. The duodenal portion 20 having a smaller diameter may also assist in insertion of the prosthesis 10, by allowing it to pass through the pylorus 101 more easily.

The stomach portion 18 and the duodenal portion 20 may both have a diameter (e.g., maximum diameter) that is larger than a diameter of the fully opened pylorus. In one embodiment, the stomach portion 18 may have a diameter, such as a maximum diameter, that is from 15 to 45 mm, or any sub-range therein. For example, the stomach portion 18 may have a diameter of 20 to 40 mm, 25 to 35 mm, or about 30 mm (e.g., .+-.5 mm). The stomach portion 18 may have a larger diameter than the duodenal portion 20 (e.g., max diameters). In one embodiment, the duodenal portion 20 may have a diameter, such as a maximum diameter, that is from 15 to 40 mm, or any sub-range therein. For example, the duodenal portion 20 may have a diameter of 15 to 35 mm, 20 to 30 mm, or about 25 mm (e.g., .+-.5 mm). The difference between the first and duodenal portion 20 diameters may be defined as a ratio. In one embodiment, a ratio of the diameter (e.g., max diameter) of the second diameter to the first diameter is less than 1:1. For example, the ratio may be less than 0.9:1, 0.8:1, 0.7:1, 0.6:1, or 0.5:1. In one embodiment, the ratio may be from 0.6:1 to 0.9:1. In another embodiment, the ratio may be from 0.7:1 to 0.9:1. In another embodiment, the ratio may be from 0.8:1 to 0.9:1. In another embodiment, the ratio may be from 0.75:1 to 0.85:1.

The plurality of strands 16 in the main body 12 may have a first, proximal end 30 and a second, distal end 32. The first and second ends of the main body 12 may be connected, attached, or otherwise coupled to the connector assembly 14. The connector assembly 14 may include two or more connectors or parts, including a proximal connector 34 and a distal connector 36. The proximal connector 34 may be spaced apart and configured to receive, couple, or attach to the first end 30 of the plurality of strands 16. The first end 30 of the strands 16 may extend in a proximal or antegrade direction from the stomach portion 18 of the main body 12 to couple to the proximal connector 34. The proximal connector 34 may therefore be proximal to the stomach portion 18 of the main body 12 when the device is in the deployed position and may reside in the stomach of the patient. The distal connector 36 may be configured to receive, couple, or attach to the second end 32 of the plurality of strands 16. The second end 32 of the strands 16 may extend in a distal or retrograde direction from the duodenal portion 20 of the main body 12 to couple to the distal connector 36. The distal connector 36 may therefore be distal to the duodenal portion 20 of the main body 12 when the device is in the deployed position and may reside in the duodenum of the patient.

The connectors may have a generally circular cross section transverse to their longitudinal axes. The proximal and distal connectors may each have a central channel or lumen 40 defined therein, which may be configured to allow chyme to flow through the device, as well as facilitate insertion and/or removal of the device. The proximal and distal connectors may each have a width or diameter that is less than the maximum diameters of the stomach portion 18 and the duodenal portion 20 of the main body 12. Accordingly, the main body 12 may have a reduced diameter portion 22 in the region where the first and second ends of the strands 16 attach to the proximal and distal connectors. In one embodiment, the main body 12 diameter may be at its minimum in the region where it attaches to the proximal and/or distal connector. The diameter of the main body 12 in the region where it attaches to the proximal and/or distal connector may be the same or similar to the diameter of the main body 12 in a region between the stomach portion 18 and the duodenal portion 20. This region may be referred to as the valley between the stomach portion 18 and the duodenal portion 20 and may be the portion that is located within the pylorus when the device is deployed.

The connector assembly 14 may also include a middle connector 42 or middle portion 42. The middle connector 42 may extend at least partially between the proximal and distal connectors. In one embodiment, the middle connector 42 is not connected or attached to the main body 12. In the deployed configuration, the middle connector 42 may be located completely within the strands 16 of the main body 12 or surrounded by the strands 16. The middle connector 42 may be coupled at its proximal end to the proximal connector 34. The middle and proximal connectors may be coupled in any suitable manner. In one embodiment, the middle and proximal connectors are coupled via a threaded engagement 44. The middle connector 42 may include male threads 46 that are configured to engage female threads 48 defined in the proximal connector 34. However, the threading may also be reversed, such that the middle connector 42 includes female threads and the proximal connector 34 includes male threads.

In at least one embodiment, the threaded engagement between the middle and proximal connectors is relatively coarse, or has a large pitch (e.g., fewer threads per axial distance). The threaded engagement may be a single start thread or a multiple start thread (e.g., two start or three start). In one embodiment, the male threads (e.g., on the middle connector 42) may have a pitch of 2 to 8 mm, or any sub-range therein. For example, the pitch may be from 3 to 7 mm, 3.5 to 6 mm, or about 4.2 mm (e.g., .+-.0.5 mm). The thread may have any suitable diameter, such as 0.635 cm (0.25 inch) to 1.27 cm (0.5 inch) or about 0.9525 cm (0.375 inch) (e.g., +/- 0.254 cm (i.e. +/- 0.1 inch)). A large pitch, and therefore a large angle of repose, may allow the middle and proximal connectors to disengage or decouple more easily than a small pitch. The angle of repose may also be referred to as the angle of friction, and generally refers to the maximum angle at which a load can rest motionless on an inclined plane due to friction, without sliding down. In one embodiment, the angle of repose of the threaded engagement may be from 3 to 15 degrees, or any sub-range therein, such as 4 to 12 degrees, 5 to 10 degrees, or about 8 degrees (e.g., .+-.2 degrees). Additional properties that may affect the disengagement of the threads may include the lubricity and the smoothness of the connectors. In one embodiment, all of the connectors in the connector assembly 14 may be formed of a plastic, such as ABS, nylon, acetyl, Teflon, PP, or PE. Plastics generally have a high lubricity with each other and may allow the threads to disengage. In another embodiment, one or more of the connectors may be formed of metal, such as stainless steel. For example, the middle connector 42 may be partially or fully formed of a metal and the proximal and distal connectors may be formed of plastic. Metals and plastics generally have a high lubricity with each other and may allow the threads to disengage.

In order to prevent relative movement or unthreading between the middle and proximal connectors when the device is deployed, a release mechanism 50 may be provided to control the disengagement of the connectors. The release mechanism 50 may be configured to prevent relative movement of the connectors until the release mechanism 50 is activated or actuated. The release mechanism 50 may be any device capable of switching between a locked or unactuated position, in which the threads are prevented from disengaging, and an unlocked or actuated position, in which the threads are free to disengage. In one embodiment, the release mechanism 50 may include a pin or rod 52. The proximal and middle connectors may each include a groove or channel 54 that extends through their threads. When the connectors are coupled together via threads, the channels 54 may cooperate to form a passage 56 that is configured and sized to receive the pin 52. Accordingly, when the pin 52 is inserted into the passage 56, the threads of the proximal and middle connectors are locked together and cannot be unscrewed. When the pin 52 is not inserted in the passage 56, the threads are able to be unscrewed. The pitch of the threads may be configured to allow the proximal and middle connectors to be unscrewed with relatively little force being applied when the pin 52 is not inserted.

The middle connector 42 may include at least one projection 58 extending from its proximal end 60 toward its distal end 62. There may be two, three, four, or more projections, for example, 2 to 10, 2 to 8, 2 to 6, or 2 to 4 projections. The projections may be radially spaced to form a channel or passage 64 extending from the proximal connector 34 towards the distal connector 36. Each projection 58 may include a snap fit element or barb 66, which may be located at a distal tip 68 of the projection. The snap fit elements may include a stop 70 extending perpendicular or substantially perpendicular to the long-axis of the projection 58 and radially outward. The snap fit elements may also include a ramp 72 extending at an angle from the stop 70 to a tip of the projection. The projections 58 may be formed of a resilient material that can deform or deflect from its original position and return to its original position.

The snap fit elements of the middle connector 42 may be configured to engage a flange or lip 74 of the distal connector 36 when the device is in the deployed position. The flange or lip 74 may be annular or extend around a perimeter of the distal connector 36. The flange or lip 74 may also be continuous around the perimeter or may have gaps or interruptions. The stops 70 of the snap fit elements may engage the flange or lip 74 when the device is in the deployed position and prevent the distal and middle connectors from being pulled away from each other. Accordingly, when the device is in the deployed position, the proximal, middle, and distal connectors may be coupled together such that the proximal and distal connectors cannot move axially apart or away from each other. The proximal and middle connectors may be coupled by a threaded engagement and the middle and distal connectors may be coupled by snap fit elements of the middle connector 42 engaged with a flange on the distal connector 36.

The distal connector 36 may include a threaded portion 76 to facilitate insertion, movement, or alteration of the device. The threaded portion 76 may include male or female threading. The threaded portion 76 may be integral to the distal connector 36 or it may be a separate component that is attached or coupled to the distal connector 36 (e.g., by adhesive or welding). The channel or lumen of distal connector 36 may extend through the threaded portion 76 such that partially digested food passes through the threaded portion 76 when the device is deployed.

The device may further include a sleeve 78 configured to extend into the duodenum and, in some embodiments, into the proximal jejunum. The sleeve 78 may be formed of a biocompatible polymer and may be impermeable or semi-permeable with respect to partially digested food and stomach fluids that are passed from the stomach to the small intestine. The sleeve 78 may be hollow, such that a lumen or passage is formed from a proximal end of the sleeve connected to the device to a distal end of the sleeve. The proximal end 80 of the sleeve may be attached to the distal connector 36. The attachment may be rigid or fixed, such that removal of the device requires removal of the sleeve, and vice versa. For example, the sleeve may be attached by adhesive (e.g., glue) or welded to the distal connector 36. The sleeve may connect to the distal connector 36 such that it surrounds an exit of the lumen in the distal connector 36. The sleeve may be dip or blow molded from one of several polymers, such as PTFE (Teflon), polyurethane or silicone.

Accordingly, partially digested food may travel from the stomach, through the lumens of the proximal connector 34 and the distal connector 36, through the sleeve, and exit in a distal portion of the duodenum or in the jejunum. The sleeve therefore is configured to reduce or eliminate the absorption of nutrients in the duodenum and proximal jejunum (depending on sleeve length), thereby reducing the number of calories absorbed by the patient.

Since the device may be deployed over a relatively long time period, it may be important to minimize or prevent tissue in-growth into the main body 12. Tissue in-growth may inhibit removal of the device and may cause removal to be traumatic to the tissue in and around the pylorus. In at least one embodiment, the spaces between the strands 16 in the main body 12 may be blocked or filled to prevent tissue in-growth. In one embodiment, the main body 12 may be partially or completely surrounded by a sheath, which may be formed of a polymeric material, such as an elastomer (e.g., silicone). The sheath may surround at least the stomach portion 18 and the duodenal portion 20 of the main body 12, and may cover all externally exposed strands 16. By covering the strands 16 of the main body 12, tissue in-growth may be prevented and the device may remain detached from the stomach, pylorus, and duodenum of the patient. The sheath may be flexible and elastic enough that it conforms to the outer shape of the main body 12 in both the deployed and collapsed configurations (explained in more detail below).

In another embodiment, the strands 16 of the main body 12 may be partially or completely embedded within a polymeric material, such as an elastomer (e.g., silicone). In this embodiment, the strands 16 are not covered on one side or surface, but encapsulated by the polymeric material such that the strands 16 are not exposed to the environment/surroundings at all. Embedding the strands 16, or at least a portion of the strands 16, in a polymeric material may minimize or prevent tissue in-growth, as described above, as well as provide additional resistance to corrosion. While an outer sheath may protect the strands 16 from exterior corrosive substances, the strands 16 may still be exposed on an interior of the main body 12. Embedded strands 16 may be isolated from corrosive substances, such as stomach acids, both external and internal to the main body 12. In one embodiment, the strands 16 may be embedded in the polymeric material (e.g., silicone) by inflating a balloon inside the main body 12 and dipping the main body 12 in liquid silicone. However, any suitable method of embedding the strands 16 in the polymeric material may be used. The polymeric material may be flexible and elastic enough that it conforms to the shape of the main body 12 in both the deployed and collapsed configurations (explained in more detail below).

When the device is deployed across the pylorus of a patient, a lumen or channel 84 may be formed from the stomach, through the proximal, middle, and distal connectors (the connector assembly 14), and into the sleeve (or into the duodenum if there is no sleeve). Partially digested food (e.g., chyme) may therefore travel through the lumen 84 in the device in a manner similar to the pylorus (e.g., without the device). Reducing the flow of chyme from the stomach into the intestines, and thereby slowing the rate of gastric (stomach) emptying, may result in weight loss in a patient. By increasing the time for the stomach to empty, the patient feels full, or satiated, for longer. This prolonged feeling of fullness reduces the desire to eat, which may result in fewer calories being consumed.

In at least one embodiment, the lumen 84 of the connector assembly 14 may be sized and configured to reduce the flow of partially digested food from the stomach to the small intestine. The lumen 84 may have a diameter that is smaller than a diameter of the pylorus, thereby increasing the resistance to the flow of chyme and slowing gastric emptying. The lumen 84 may have a constant, or substantially constant, diameter or the diameter may vary along a length of the lumen 84. The diameter of the lumen 84 may be smaller than a diameter of the pylorus in at least one region of the lumen 84. For example, the lumen 84 may be smaller than a diameter of the pylorus within the proximal connector 34 channel, within the middle connector 42, and/or within the distal connector 36 channel. The lumen 84 may be narrower than the pylorus in more than one region and it may be as wide as the pylorus in some regions. In one embodiment, the lumen 84 may be narrowest within the distal connector 36 channel.

The more resistance to flow that is created, the slower the gastric emptying will be, and the more weight loss should occur. Accordingly, the size of the lumen 84 may be designed or configured based on the level of obesity in the patient being treated. For morbidly obese patients, the lumen size may be made smaller than for a slightly or moderately obese person. Accordingly, the size of the lumen 84 and the aggressiveness of the weight loss goal can be tailored to each patient, depending on their situation and needs. The typical pyloric diameter has been measured to be from about 7 mm to about 10 mm. In one embodiment, at least a portion of the lumen 84 of the device may be from about 3 mm to about 7 mm, or any sub-range therein. For example, the lumen size may be about 4 mm to about 6 mm or about 5 mm +-.0.5 mm. The lumen size may be adjusted by changing the channel size of the proximal and/or distal connector 36.

The device may be deployed or inserted into the patient through the mouth and the esophagus and into the stomach. Since the device is inserted through the mouth, the procedure may be performed using endoscopic or radiological guidance. The device may include radiological markers (not shown) to facilitate insertion using fluoroscopy. The procedure may also be an outpatient procedure, making it less expensive and less traumatic for the patient.

The following figures depict alternative embodiments of a pyloric implant device. In some or all of the following embodiments, a connector assembly might not be included. Thus, these embodiments may be analogous to the main body portion of the pyloric implant described above. Other embodiments may include a connector assembly or other mechanisms to facilitate delivery of the device into the pylorus. In general, each embodiment of the pyloric implant described below includes at least one support member (or "frame member") and a material disposed over the support member. Each embodiment of the pyloric implant also includes a wide stomach anchoring portion, a wide duodenal anchoring portion and a narrower pyloric spanning portion between the two. Some embodiments include a restrictor, which is attached to the support or frame member. In other embodiments, the restrictor is simply part of the support or frame member, shaped in such a way that it slows passage of food out of the stomach through the pylorus. As mentioned previously, the restrictor or restrictive portion of the device may be located anywhere along the pyloric portion. As also mentioned previously, any restrictor described below may be used with any version of the pyloric device and in different combinations. In other words, features described below in reference to one embodiment may be incorporated into any other embodiments.

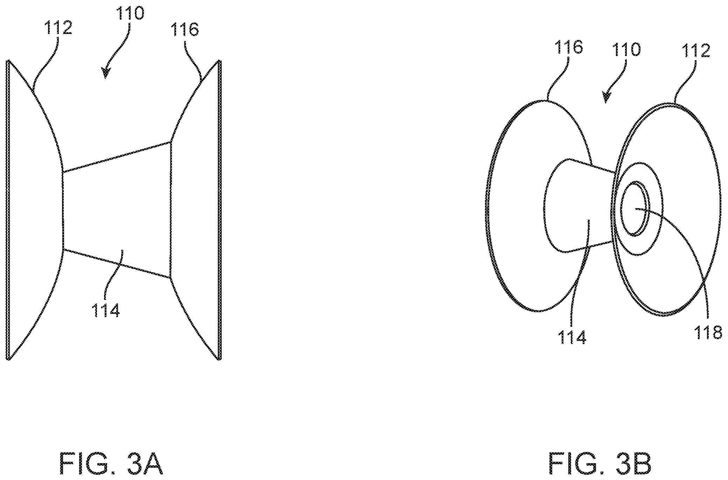

Referring now to Figs. 3A and 3B, one embodiment of a pyloric implant 110 is illustrated. In these figures, only the outer material of the pyloric implant 110 is shown, to illustrate the overall shape of the prosthesis 110. The inner support member (or multiple support members, in some cases) is not shown. As is immediately apparent, the overall shape of the pyloric implant 110 is different than the two-disc shape of the previously described embodiment. In the present embodiment, the prosthesis 110 includes a stomach portion 112, a pyloric portion 114, and a duodenal portion 116. A channel 118 extends through the pyloric portion 114 to allow for the passage of food out of the stomach and into the duodenum. In this embodiment, the stomach portion 112 and the duodenal portion 116 both have slightly curved walls and are both the same (or approximately the same) diameter. The pyloric portion 114 is tapered, with a smaller diameter at its junction with the stomach portion 112, expanding to a wider diameter at its junction with the duodenal portion 116. The effect of this tapered shape is to restrict passage of food through the channel 118 to only pieces that are small enough to fit through the opening at stomach end of the device 110. Restricting passage at the stomach portion 112 prevents food from getting stuck in the pyloric portion 114, which could lead to obstruction. This embodiment does not include an additional restrictor at the junction of the pyloric portion 114 with the stomach portion 112, but such restrictors will be described further below in relation to alternative embodiments.

The overall shape and size of the pyloric implant 110 of Figs. 3A and 3B is merely one example, and additional examples will be described below. In various embodiments, any number of features, shapes and sizes may be changed. For example, the walls that make up the stomach portion 112 and the duodenal portion 116 may be curved (as shown) or straight (i.e., cone shaped). They may have the same diameter or different diameters. The pyloric portion 114 may be tapered or straight and may have any of a wide range of lengths and diameters. Similarly, the channel 118 extending through the pyloric portion 114 may have any of a wide range of diameters. Any one or more of these shape characteristics may be adjusted to form a new embodiment. The material used to form the prosthesis 110 may be any suitable, biocompatible material for use in this application, for example polytetrafluoroethylene (PTFE) or any other material commonly used in the manufacture of stent grafts or other implantable devices for use in the body. It may be molded or otherwise formed in the shape of the prosthesis 110, and the material is typically resilient, so that if it is collapsed into a small-diameter configuration for delivery through a delivery device, it expands upon deployment to its default shape.

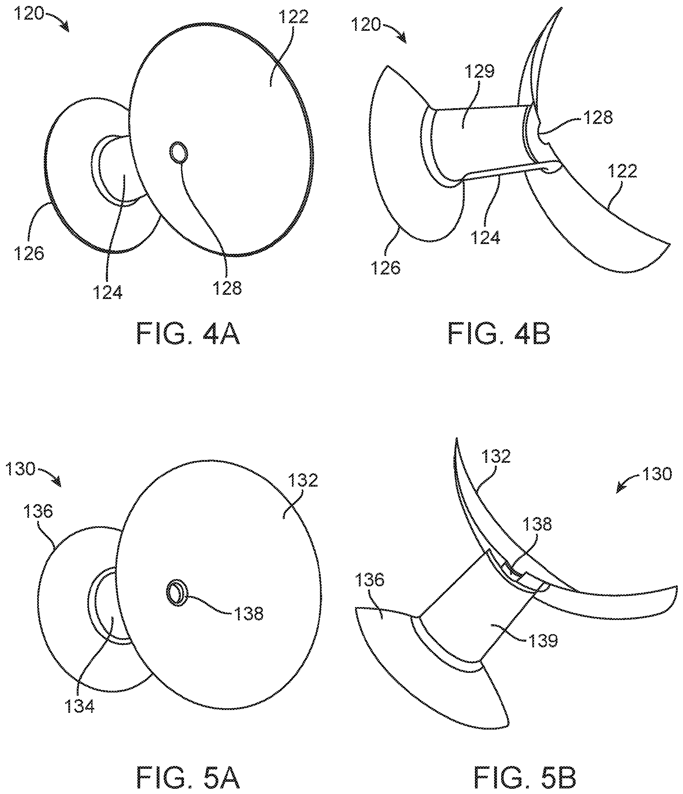

Figs. 4A and 4B illustrate another embodiment of a pyloric implant 120. In this embodiment, the stomach portion 122 has a larger maximum diameter than the duodenal portion 126. The pyloric portion 124 is straight (or nearly straight), rather than tapered, and a restricting aperture 128 is included as part of the stomach portion 122, leading into the channel 129 of the pyloric portion 124. The aperture 128 has a diameter much smaller than the inner diameter of the pyloric portion 124, so that any food material small enough to pass through the aperture 128 should pass easily through the channel 129. In an alternative embodiment, the pyloric portion 124 may also be tapered.

Figs. 5A and 5B show another embodiment of a pyloric implant 130, which again includes a stomach portion 132, a pyloric portion 134 and a duodenal portion 136. In this case, a small restrictor 138 is formed in the middle of the stomach portion 132 to form the opening from the stomach into the channel 139 of the pyloric portion 134. Alternatively, the restrictor 138 may be a separate piece that is attached within a hole in the center of the stomach portion 132. The restrictor 138 functions just as the aperture 128 in the previous embodiment. Again, the restrictor 138, the aperture 128 and any similar restrictors or apertures described below may be positioned at the stomach end of a pyloric implant, at the duodenal end, or anywhere in between. This will not be repeated for every embodiment described below and is applicable to all embodiments.

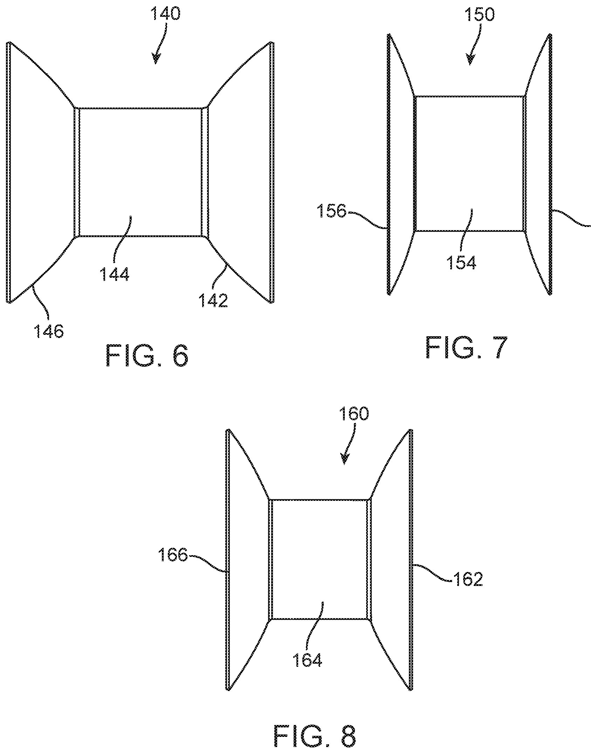

Referring now to Figs. 6-8, three different embodiments of a pyloric implant 140, 150, 160 are shown, illustrating that the various parts of the implant 140, 150, 160 may have any of a number of suitable shapes and sizes. In the embodiment of Fig. 6, the pyloric implant 140 includes a stomach portion 142, a pylorus spanning portion 144 and a duodenal portion 146. The stomach portion 142 and the duodenal portion 146 each have a length, a curvature (or no curvature in some embodiments), a minimum diameter and a maximum diameter, and each forms an angle with the outer surface of the pylorus spanning portion 144. In the embodiment shown, all of these characteristics are the same, or approximately the same, for the stomach portion 142 and the duodenal portion 146. In alterative embodiments, any of these characteristics may be altered, and the stomach portion 142 and the duodenal portion 146 may have any number of different measurements and configurations. For example, as already mentioned, the stomach portion 142 may have a larger maximum diameter than the duodenal portion 146. Similarly, the pylorus portion 144 may have any suitable length, shape and diameter. For example, it may be straight, as in Fig. 6, or it may be tapered, with a smaller diameter adjacent the stomach portion 142 and a larger diameter adjacent the duodenal portion 146, or vice versa. Any shape and size changes may be made to the embodiments described herein, according to various alternative embodiments.

Referring to Fig. 7, a differently sized and shaped pyloric implant 150 also includes a stomach portion 152, a pyloric portion 154 and a duodenal portion 156. Compared to the previous embodiment, the pyloric portion 154 of this implant 150 is shorter and has a wider diameter. The stomach portion 152 and the duodenal portion 156 are each shorter and form a steeper angle with the outer surface of the pyloric portion 154. Similar to the previous embodiment, the stomach portion 152 and the duodenal portion 156 are slightly curved, whereas in alternative embodiments they may be either straight or more curved.

Fig. 8 illustrates yet another embodiment of a pyloric implant 160, including a stomach portion 162, a pyloric portion 164 and a duodenal portion 166. In this embodiment, the stomach portion 162 and the duodenal portion 166 have lengths that are between the lengths of the corresponding portions in the previous two embodiments. The pyloric portion 164 is relatively short and has a larger outer diameter.

As can be seen from the three embodiments of Figs. 6-8, each portion of a pyloric implant 140, 150, 160 may have any suitable shape and size, such as but not limited to straight or conical and/or having proximal and distal portions with differing diameters. For example, in various embodiments, a stomach portion may have a maximum diameter of about 25 mm to about 50 mm, a pyloric portion may have an inner diameter of about 8 mm to about 15 mm, and a duodenal portion may have a maximum diameter of about 15 mm to about 35 mm. The length of the pyloric portion may vary from about 10 mm to about 20 mm, and the overall length of the implant may range from about 25 mm to about 50 mm. The opening leading into the pyloric portion (at the junction of the pyloric portion and the stomach portion) may have a minimum diameter of about 3 mm to about 5 mm and a maximum diameter of about 8 mm to about 15 mm, in various embodiments. The opening at the opposite end of the pyloric portion, where it joins the duodenal portion, may have a diameter of about 8 mm to about 15 mm. These size ranges are only examples and should not be interpreted as limiting the scope of the invention.

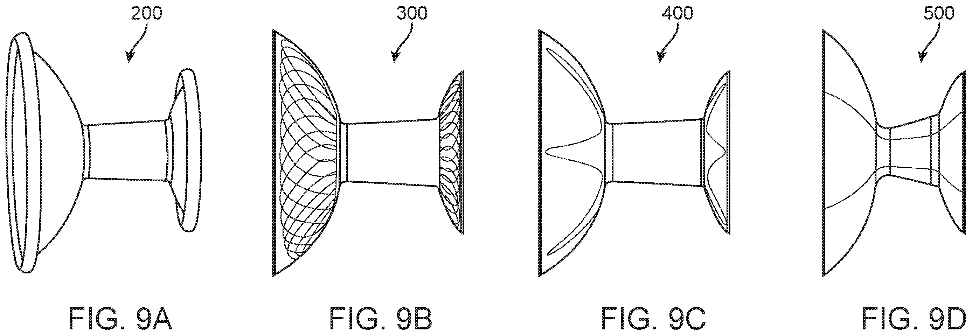

Referring next to Figs. 9A-9D, embodiments of the pyloric implant described herein generally include one or more frame or support components and one or more layers of material disposed over/around the frame/support component(s). In many embodiments, for example, one or more support members are sandwiched between two layers of material. This is analogous to a stent graft, where the stent acts as the frame and the graft acts as the material. Typically, the stomach portion and the duodenal portion will include some type of support/frame structure, while the pyloric portion may or may not include support elements. Providing a very flexible pyloric portion allows the natural pylorus freedom to open and close to perform its natural valve function. In alternative embodiments, however, either of the stomach or duodenal portions may not include a frame component, and/or the pyloric portion may include some type of frame component. Although an almost infinite number of variations of frames and materials may be used in various embodiments, four general embodiment types are illustrated in Figs. 9A-9D and described in greater detail in subsequent figures.

Pyloric implant embodiment type one 200 is illustrated in Fig. 9A. The frame components in this embodiment are at least two rings, which may be disposed on the stomach and duodenal portions, at opposite ends of the implant 200 or elsewhere along its length, as will be described further below. Fig. 9B illustrates pyloric implant embodiment type two 300, in which the support includes at least one braided element embedded in the material of the stomach portion and the duodenal portion. Referring to Fig. 9C, pyloric implant embodiment type three 400 uses a star-shaped frame in each of the stomach and duodenal portions. Finally, as shown in Fig. 9D, pyloric implant embodiment type four 500 includes a continuous wire support member that extends the length of the implant 500, then proceeds along the edge and doubles back toward the other side of the implant 500. All four general types of pyloric implant embodiments will be described in further detail below.

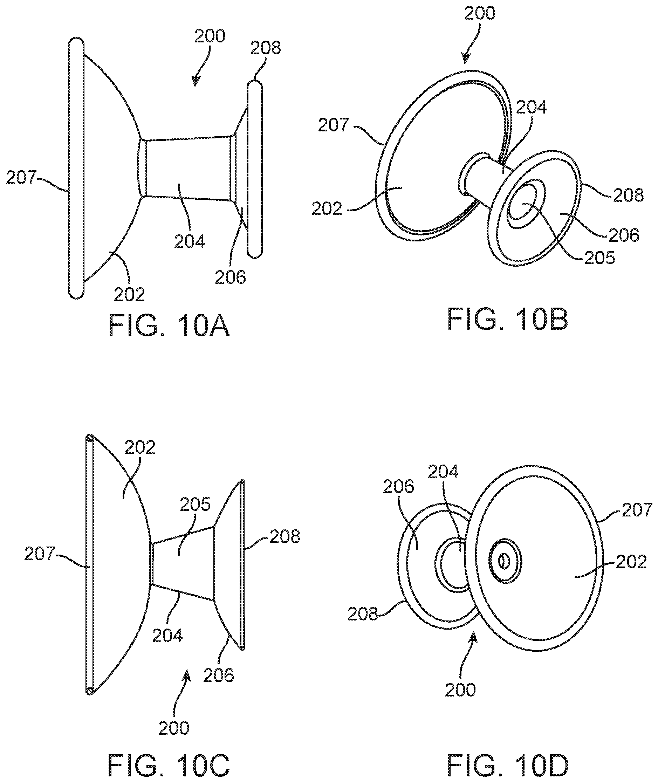

With reference now to Figs. 10A-10D, one embodiment of a pyloric implant 200 (general embodiment type one) is illustrated in side view (Fig. 10A), rear perspective view (Fig. 10B), side cross-sectional view (Fig. 10C), and front perspective view (Fig. 10D). In this embodiment, the pyloric implant 200 includes a stomach portion 202, a pyloric portion 204 and a duodenal portion 206, all made of a suitable implant material, such as PTFE or the like. At opposite ends of the implant 200, embedded within the material, there is a stomach support ring 207 and a duodenal support ring 208. A channel 205 extends through the pyloric portion 204, from the stomach portion 202 to the duodenal portion 206, to allow food material to pass from the stomach to the duodenum. The support rings 207, 208 may have any suitable size and may be made of any suitable material, such as any shape memory plastic, polymer or metal. For example, the rings 207, 208 may be made of Nitinol or the same material used to make any other portion of the pyloric implant 200. As described in previous embodiments, the pyloric portion 204, and thus the channel 205, may be tapered, providing a narrower opening at the stomach portion 202 and a wider opening at the duodenal portion 206, thus preventing clogging of the channel 205 with food material. (Alternatively or additionally, the pyloric implant 200 may include a restrictor.) Optionally, one or more additional rings may be disposed within the stomach portion 202 and/or the duodenal portion 206 for additional support.

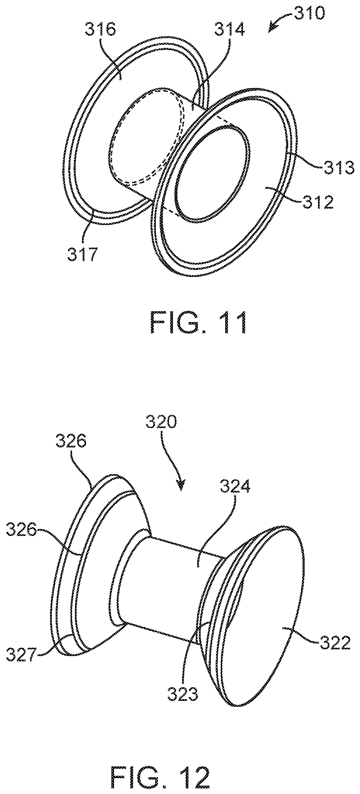

Referring now to Fig. 11, an alternative embodiment of a pyloric implant 310 (general embodiment type one) may include a stomach portion 312 with a stomach support ring 313, a pyloric portion 314, and a duodenal portion 316 with a duodenal support ring 317. Again, the support rings 313, 317 may be made of a shape memory material, such as but not limited to a shape memory metal, such as Nitinol. The rings 313, 317 not only help the material of the implant 310 resume its expanded, default shape after being delivered from a delivery device, but they also support the stomach portion 312 and the duodenal portion 316, to help them act as anchors and to prevent the pyloric implant 310 from passing out of the stomach and into the small intestine or sliding retrograde into the stomach. In this embodiment, the support rings 313, 317 are located slightly away from the extreme outer edges of the stomach portion 312 and the duodenal portion 316. In fact, in various embodiments, the rings 313, 317 may be placed at any suitable location along the length of the stomach portion 312 and the duodenal portion 316.

In Fig. 12, another embodiment of a pyloric implant 320 is illustrated. Just as with previous embodiments, this embodiment includes a stomach portion 322, a pyloric portion 324 and a duodenal portion 326. The stomach portion 322 includes a stomach support ring 323, and the duodenal portion 326 includes a duodenal support ring 327. The rings 323, 327, in this embodiment, are located closer to a midpoint along the lengths of the stomach portion 322 and the duodenal portion 326. In alternative embodiments, multiple rings may be included on the stomach portion 322, the duodenal portion 326 or both. For example, in one embodiment, first support rings may be located along the outer edge (or "rim") of the stomach portion and the duodenal portion, and second support rings may be located somewhere along the lengths of both portions. Any suitable number and placement of rings may be used, in various alternative embodiments.

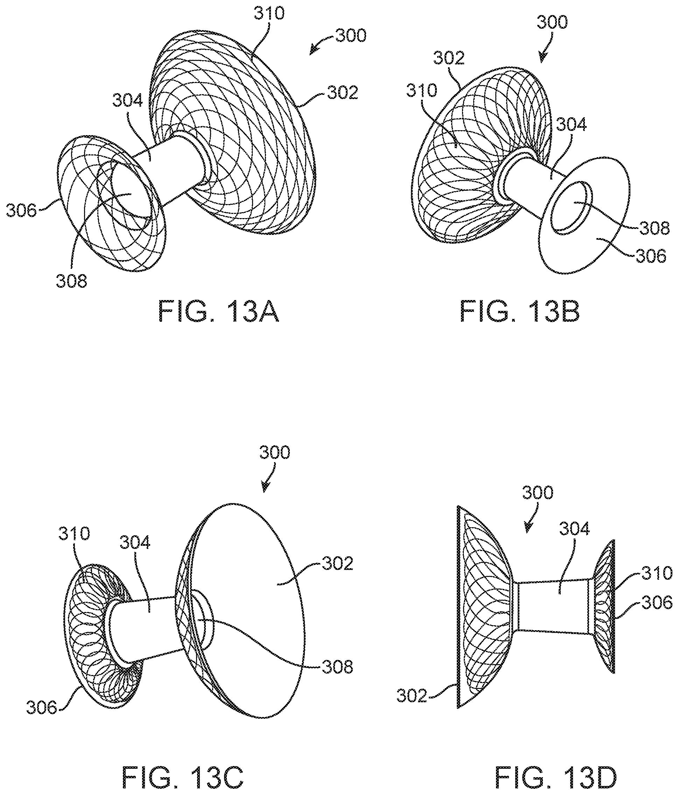

Referring now to Figs. 13A-13D, another alternative embodiment of a pyloric implant 300 is illustrated. This implant 300 is of the general embodiment type two, which includes one or more braided support members. In this embodiment, the stomach portion 302 and the duodenal portion 306 of the pyloric implant 300 each include an embedded braided support 310, which may be made of any suitable material, such as shape memory metal, and which may have any suitable braiding pattern, tightness and overlap of braiding material, etc. Generally, the braided supports 310 are configured to change from a collapsed configuration, for delivery through a catheter delivery device, to an expanded configuration, as shown. Although the braided supports 310 are shown in Figs. 13A-13D for clarity of illustration, in at least one embodiment of the actual pyloric implant 300, they are sandwiched between two layers of the material that makes up the rest of the implant 300. In some embodiments, the braided supports 310 may be visible through transparent or translucent material, while in other embodiments they may not be visible from the outside of the implant 300. In this embodiment of the implant 300, the stomach portion 302 has a larger maximum diameter than the duodenal portion 306, and both portions are curved. The pyloric portion 304 is tapered, with a narrower end residing at its junction with the stomach portion 302. In alternative embodiments, the stomach portion 302 and/or the duodenal portion 306 may be straight, rather than curved, and/or the pyloric portion 304 may be non-tapered. In some embodiments, the implant 300 may also include any of the restrictors described in this application.

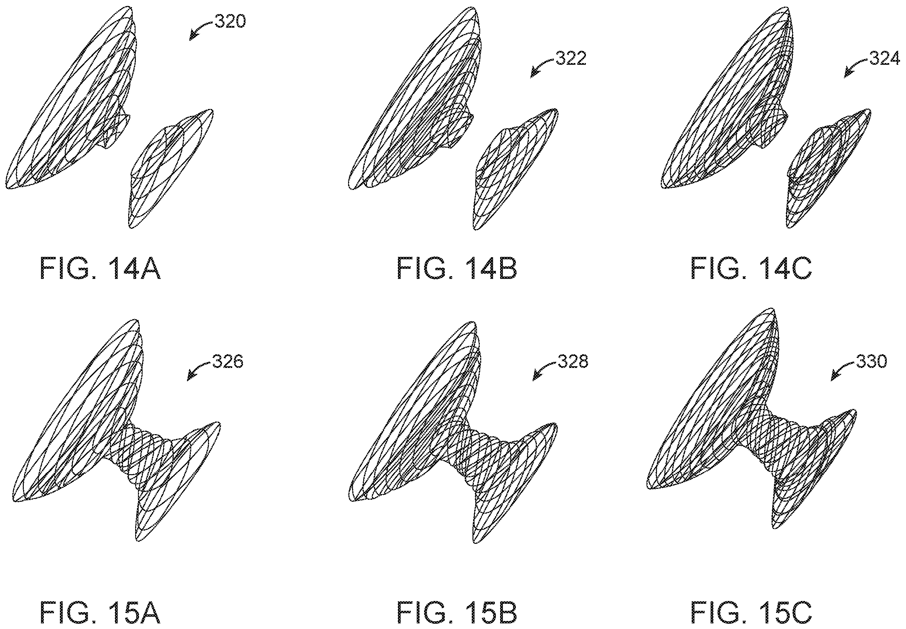

Figs. 14A-14C illustrate three different embodiments of configurations for braided wire support members 320, 322, 324. All three embodiments include two support members each, one for the stomach portion and one for the duodenal portion of a pyloric implant. In the first embodiment (Fig. 14A), the wire braiding of the support members 320 is relatively loose. The support members 322 of Fig. 14B have a slightly tighter weave, and the support members 324 of Fig. 14C have an even tighter weave.

Referring to Figs. 15A-15C, three alternative braided support members 326, 328, 330 are shown. Each of these embodiments includes one continuous support member 326, 328, 330 that extends from the stomach portion, through the pyloric portion and onto the duodenal portion of the pyloric implant. As with the previous three figures, the weave is loosest in the support member 326 of Fig. 15A, is slightly tighter in the support member 328 of Fig. 15B, and is tightest in the support member 330 of Fig. 15C. In alternative embodiments, any other suitable weave patterns and braiding densities may be used. Different materials and/or thicknesses of braiding materials may also be used in different embodiments. Some embodiments may include more than two support members, and the various support members may be positioned at any location or combination of locations along the pyloric implant.

In various alternative embodiments of the support members 326, 328 and 330, the braided material used to make the proximal portion and the distal portion may be different from the braided material used to make the pyloric portion. For example, the material of the proximal and distal portions may be a shape memory material, such as Nitinol or other shape memory metals, while the material of the pyloric portion may be a suture material, fabric or textile. This combination may be advantageous, as it provides for the springing open of the proximal portion and the distal portion upon delivery out of a delivery device, while also providing a softer material for spanning the pylorus. Suture material, for example, provides structural support to the pyloric portion, while allowing the pylorus to contract and work properly and without being so rigid as to potentially damage the pylorus.

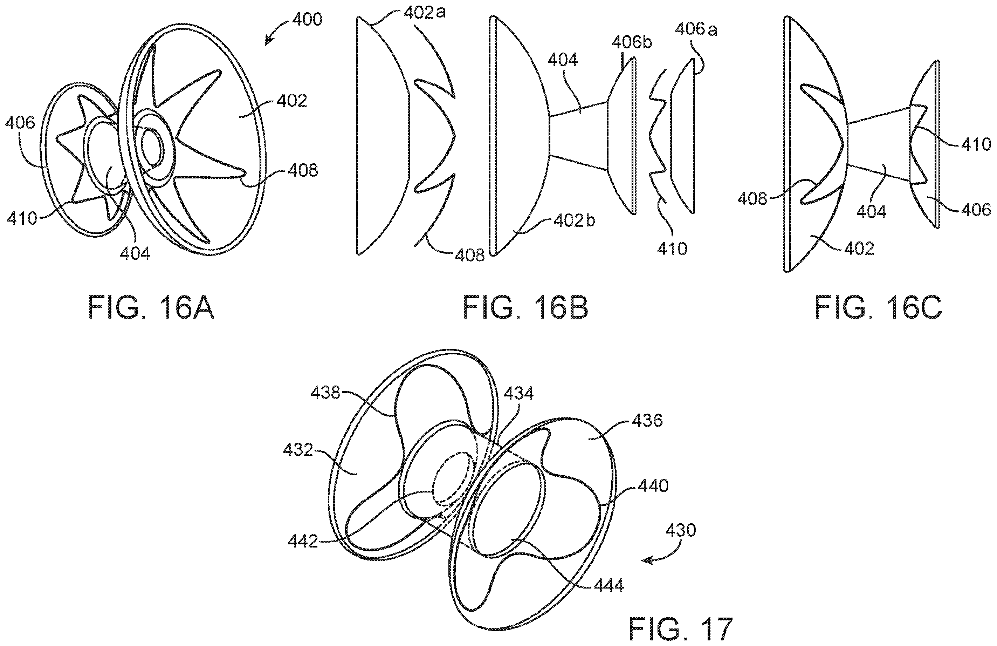

With reference now to Figs. 16A-16C, another embodiment of a pyloric implant 400 is illustrated in perspective view (Fig. 16A), exploded view (Fig. 16B) and side view (Fig. 16C). This embodiment of the pyloric implant 400 is in the general embodiment category type three, which includes some type of non-braided support member in each of the stomach portion 402 and the duodenal portion 406. This embodiment of the implant 400 includes a stomach portion 402, a pyloric portion 404, a duodenal portion 406, a star-shaped stomach support member 408, and a star-shaped duodenal support member 410. The pyloric portion 404 is tapered, to prevent food from clogging the pyloric portion 404. This embodiment may also include a restrictor (not shown). Fig. 16B illustrates that the stomach support member 408 is located between a first stomach portion layer 402a and a second stomach portion layer 402b. Similarly, duodenal support member 410 is located between a first duodenal portion layer 406a and a second duodenal portion layer 406b. In an alternative embodiment, the two layers may encompass the entire implant, so that the implant includes two full layers of material, from the stomach end to the duodenal end, with the support members 408, 410 sandwiched between them. The layers 402a, 402b, 406a, 406b may be joined by any suitable manufacturing means, such as by adhesive. Each of the support members 408, 410, which may be made of any suitable shape memory metal or other material, may have any number of points and may extend all the way to the edges of the stomach portion 402 and the duodenal portion 406 or may be positioned slightly away from the edges, as shown. The shape of the support members 408, 410 may be altered as well, with the star shapes having sharper or less sharp points, the stars having any number of points, and the like.

Fig. 17 illustrates an alternative embodiment of a type three pyloric implant 430. As with previous embodiments, the pyloric implant 430 includes a stomach portion 432, a pyloric portion 434 and a duodenal portion 436. Here, the stomach support member 438 and the duodenal support member 440 are shaped as looser, more rounded, three-point "stars" or flowers, with the support members 438, 440 extending almost to the inner and outer edges of the stomach portion 432 and the duodenal portion 434. Again, according to various embodiments, the support members 438, 440 may take any suitable shape and size.

This embodiment of the pyloric implant 430 illustrates another important optional configuration for limiting food passage through the pylorus. In this embodiment, the pyloric portion 434 is straight or approximately straight, the stomach portion 432 forms a small aperture 442, and the duodenal portion 436 forms a large aperture. Thus, similar to tapered embodiments, the pyloric implant 430 restricts the passage of food through the pyloric portion 434 to only pieces that are small enough to fit through the small aperture 442. This variance in size between the small aperture 442 and the large aperture 444 should prevent food from getting clogged in the pyloric portion 434.

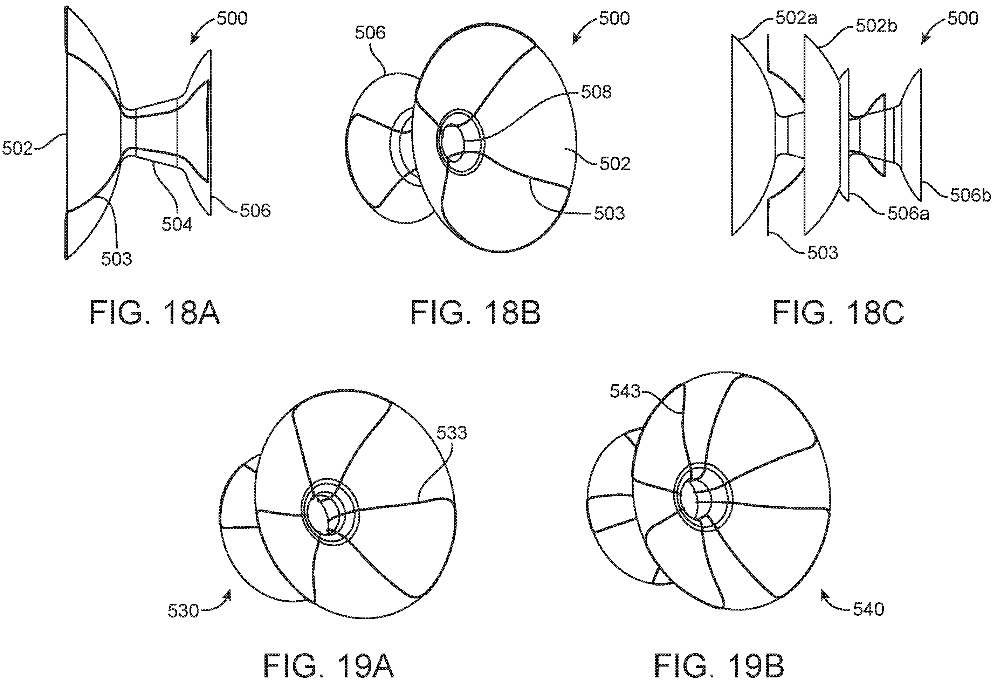

Referring now to Figs. 18A-18C, another embodiment (general embodiment type four) of a pyloric implant 500 is illustrated in side view (Fig. 18A), perspective view (Fig. 18B) and exploded view (Fig. 18C). This type of embodiment includes a continuous wire support member 503, which extends the length of the implant 500. (Alternative embodiments may include multiple continuous wire supports.) In this embodiment, the pyloric implant 500 includes a stomach portion 502, a pyloric portion 504, a duodenal portion 506 and one continuous wire support member 503. In this embodiment, the pyloric portion 504 is tapered, but in alternative embodiments it may be straight and include a restrictor. Fig. 18C shows how the wire support member 503 is positioned between a first stomach portion layer 502a and a second stomach portion layer 502b, as well as a first duodenal portion layer 506a and a second duodenal portion layer 506b. The continuous wire support member 503 is configured to extend along a portion of a rim of the stomach portion 502, then angle toward and through the pyloric portion 504, all the way to the rim of the duodenal portion 506. The wire support member 503 then extends along a portion of the rim of the duodenal portion 506 and angles back toward the pyloric portion 504 and through to the rim of the stomach portion again. Any given embodiment may have any suitable number of turns and passes across the implant 500. As best seen in Fig. 18B, the present embodiment includes two "petals," as in a flower, on the stomach portion 502 and also on the duodenal portion 506. Alternative embodiments may include any alternative number of petals.

Referring to Figs. 19A and 19B, two alternative embodiments of a pyloric implant 530, 540 are illustrated. In the embodiment of Fig. 19A, the pyloric implant 530 includes a continuous wire support member 533 with three petals (or "folds"), instead of the two petals of the previous embodiment. In the embodiment of Fig. 19B, the pyloric implant 540 includes a continuous wire support member 543 with four petals. Alternative embodiments may include any suitable number of petals or folds in the continuous wire support member.

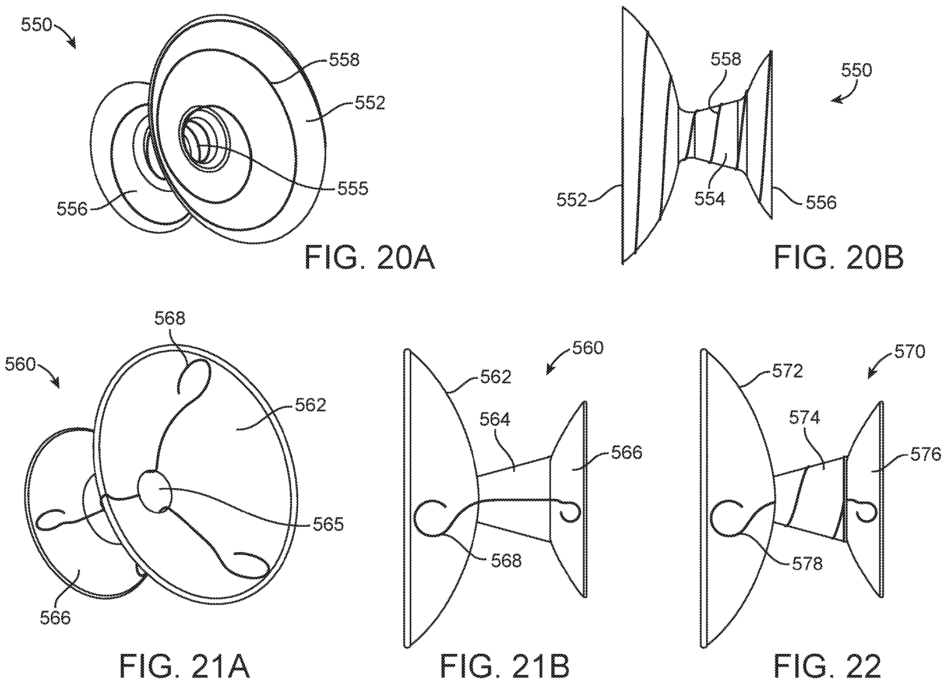

With reference now to Figs. 20A and 20B, an alternative embodiment of a pyloric implant 550 may include a differently configured continuous wire support member 558. This embodiment includes a stomach portion 552, a tapered pyloric portion 554 that forms a channel 555, and a duodenal portion 556. The wire support member 558 is shaped as a spiral, extending from the rim of the stomach portion 552, through the pyloric portion 554 and to the rim of the duodenal portion. The spiral shape may be as tightly wound or as loose as desired, in various embodiments.

Figs. 21A and 21B show another embodiment of a pyloric implant 560, which includes a stomach portion 562, a pyloric portion 564 and a duodenal portion 566. This embodiment includes multiple wire support members 568, each of which includes a hook at one end, on the stomach portion 562 and a hook at its opposite end on the duodenal portion 566. This embodiment includes three wire support members 568, but alternative embodiments may include any other suitable number.

Fig. 22 shows yet another embodiment of a pyloric implant 570, which again includes a stomach portion 572, a pyloric portion 574 and a duodenal portion 576. This embodiment includes a continuous wire support member 578, which has proximal and distal hooks, as in the previous embodiment, but which spirals around the pyloric portion 574, similar to the embodiment of Figs. 20A and 20B. As should be apparent from these drawing figures, any suitable configuration of wire support member(s) may be incorporated into a given embodiment of a pyloric implant.

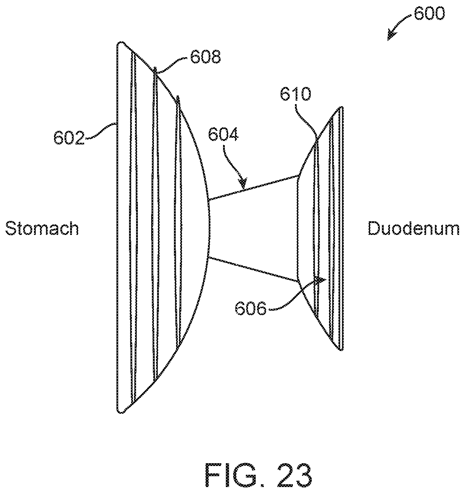

Referring now to Fig. 23, another embodiment of a pyloric implant 600 is illustrated. This embodiment includes a stomach portion 602, a pyloric portion 604 and a duodenal portion 610. Additionally, the implant 600 includes an optional feature-a stomach portion outer surface feature 608 and a duodenal portion outer surface feature 610. The surface features 608, 610 are configured to enhance the ability of the stomach portion 602 to grip/adhere to the wall of the stomach and the duodenal portion 610 to grip/adhere to the wall of the duodenum. In this embodiment, both surface features 608, 610 are a series of multiple, parallel, raised ribs. Alternative embodiments may include surface features in the form of a roughened surface, stippling, raised dots, embedded microfibers, raised asymmetrical shapes, etc. Optionally, the pyloric portion 604 may also include surface features. In fact, in any given embodiment, the stomach portion 602, the pyloric portion 604, the duodenal portion 606 or any combination thereof may include one or more surface features. Furthermore, any of the embodiments described above may include one or more surface features.

Referring further to surface features, any of the embodiments described above may be removable, repositionable, or both, sometime after implantation across the pylorus. In some cases, surface features and/or other design features of the pyloric implant may facilitate removability. For example, a thermo-reversible adhesive, which solidifies at body temperature, may be useful to initially attach a pyloric implant and subsequently reverse the attachment temporarily to readjust the implant position by flowing a cold BSS solution through the pylorus. Alternatively or additionally, an adherent including microfibers may be used. Other embodiments may include regions of stippling, which may represent application locations for a number of different potential surface adherents or adhesives. Surface adherents may provide adhesion or adherence within a relatively short period of time (e.g., less than or equal to one second, less than 1 to 5 minutes, or less than 1 to 5 hours), are reversible, and/or otherwise provide a mechanism for easily detaching a device after adhesion to the stomach, pylorus and/or duodenum. For instance, regions of stippling on the surface of a pyloric implant may include a thermo-reversible bioadhesive polymer, such as polymerized N-isopropyl acrylamide (pNIPAM). Alternatively, the regions of stippling may include a plurality of microfibers, for example, having physical surface texturing designed to mimic the feet of certain lizards and insects. In some embodiments, reversible adhesion is provided by a substance that changes its adhesion characteristic with an intensity or wavelength of light, vibration of the adhesion interface, application or concentration of a chemical substance, exposure or intensity of an electric or magnetic field, or the like.

Polymeric systems that may modify adhesive properties in response to changes in the physical and chemical characteristics of the physiological medium are promising candidates to achieve reversible tissue adhesion. For example, dynamic stimulus-responsive surface chemistries for cell patterning, thermo-active, electricalactive, and photo-active chemistries have been defined for cellular adhesion. In general, all of these chemistries operate under the same principle. These substances can be switched from a state that prevents cellular attachment to a state that promotes it. In the context of the present application, a reversible adhesive means one that can change state depending on certain stimulus, such as temperature for a thermo-reversible adhesive. Other possible stimuli include mechanical (e.g., vibration), light, radiation, and chemical.

Some embodiments of a pyloric implant may include a thermo-reversible bioadhesive polymer coating or material, such as a composition that is liquid at or below room temperature and forms a high viscosity layer or gel at body temperature. The bioadhesive, for example used as a coating on the outer surface of the implant, may help keep the implant anchored in the desired implant location, spanning from the stomach, through the pylorus and into the first part of the duodenum. In certain embodiments, the thermo-reversible bioadhesive polymer has a first adherence value when at a temperature that is a predetermined amount below an average body temperature and a second adherence value when at a temperature that is at or above the average body temperature, the second adherence value being greater than the first adherence value. The second adherence value will generally be at least twice that of the first adherence value, but may be at least 5 times, at least 10 times, or at least 100 times that of the first adherence value.

Polymers having bioadhesive properties include water-soluble cellulose derivatives, such as sodium carboxymethyl cellulose, and polyacrylic acids, which are used in many pharmaceutical preparations to improve the contact between drug and body. In some embodiments, viscosity-increasing polymers may be used, such as the cellulose derivatives, polyvinyl alcohol and polyvinylpyrrolidone. In some embodiments, gelling of a portion of the pyloric implant may be induced by an increase in the amount of electrolytes or a change in pH. Further, certain water-soluble nonionic cellulose ethers in combination with a charged surfactant and optional additives in water have the property of being liquid at room temperature and forming a gel when warmed to body temperature, and the process is reversible.

In alternative embodiments, the pyloric implant may include regions of stippling, which may be in the form of physical surface texturing designed to mimic the feet of certain lizards and insects. The stippling may be combined with microfibers that in some aspects are similar to synthetic microfibers. Microfibers may be defined as fibers having a diameter of between about 3-5 microns. The microfibers may be provided in sufficient numbers/density over a particular area of the pyloric implant to provide adhesion between the implant and the stomach, duodenum, pylorus or any combination thereof.

It is also possible to combine different surface adherents on a single embodiment of the pyloric implant, such as a bioadhesive (e.g., pNIPAM) and microfibers (e.g., gecko feet). In one embodiment, for example, microfibers on the pyloric implant may be coated with a bioadhesive that is reversible, so as to be relatively thick at room temperature and liquid at body temperature. This configuration prevents the microfibers from sticking to surrounding structures and instruments prior to implant but exposes the microfibers after implant for good adhesion or adherence within the pylorus.

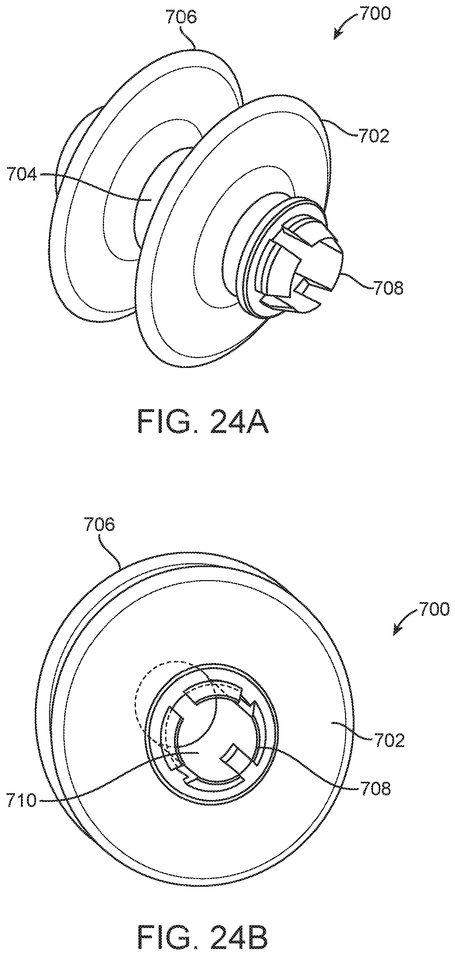

Referring now to Figs. 24A and 24B, in some embodiments, a pyloric implant 700 may include a removable restrictor (not shown), which can be attached to a restrictor attachment member 708 positioned in the opening of the implant 700 at the stomach portion 702. As with previously described embodiments, the pyloric implant also includes a pyloric portion 704 and a duodenal portion 706. As best seen in Fig. 24B, the restrictor attachment member 708 resides at the stomach-facing opening into the channel 710 that passes through the pyloric implant 700. In this embodiment, the restrictor attachment member 708 extends through, and forms the inner diameter of, the channel 710, which extends through the pyloric portion 704. The restrictor attaches to the end of the restrictor attachment member 708 and forms a smaller inner diameter at least at the opening of the channel 710. In one embodiment, for example, the restrictor may have an inner diameter of about 8.5 mm. By making the restrictor 708 removable, it is possible to adjust the speed by which the stomach can empty, thus potentially accelerating or decelerating weight loss in the patient.

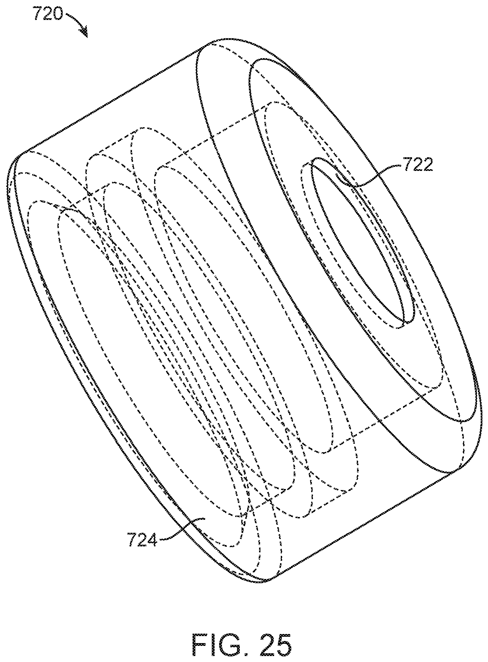

Referring to Fig. 25, one embodiment of a removable restrictor 720 for a pyloric implant is illustrated. In this embodiment, the restrictor 720 includes a narrow opening 722 at one end, which faces into the stomach, and a wide opening 724 at the opposite end, which attaches to a pyloric device, such as by the attachment to the restrictor attachment member 708 of the embodiment in Figs. 24A and 24B. The narrow opening 722 restricts passage of food through the restrictor 720, to slow gastric emptying and help prevent clogging of the pyloric implant. In alternative embodiments, the restrictor 720 may take any of a number of different configurations and sizes.

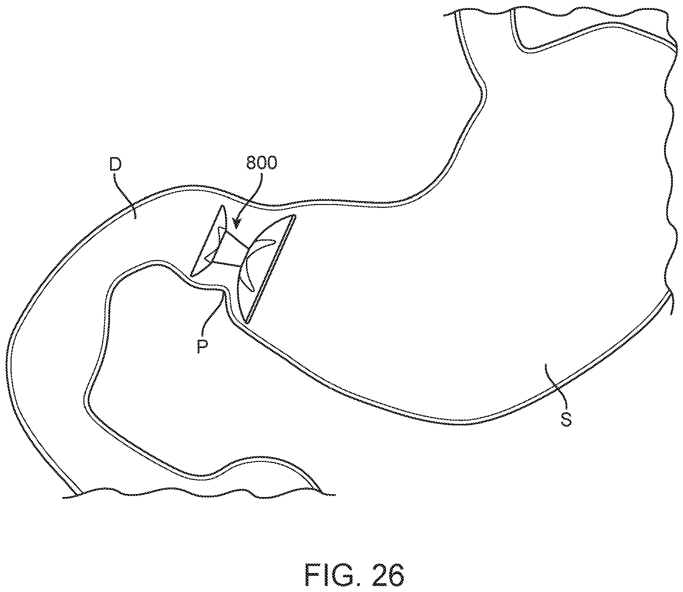

Referring now to Fig. 26, a portion of a human digestive tract, including a stomach S, pylorus P and duodenum D is illustrated in cross section. One embodiment of a pyloric device 800 is shown implanted in the pylorus P, with anchoring portions in the stomach S and duodenum D. Fig. 26 is provided to illustrate the general location at which the pyloric device 800 (and other embodiments described herein) is placed.

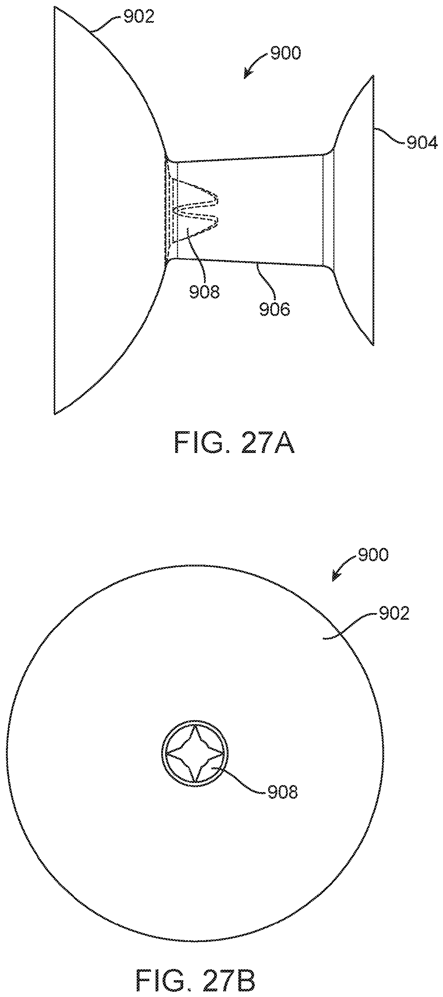

Figs. 27A and 27B illustrate another embodiment of a pyloric device 900, in side view and front view, respectively. In many of the embodiments described above, the pyloric devices include a restrictor or a restricting feature that maintains a constant inner diameter, regardless of whether food is passing through it, pushing against it, etc. These restrictors may be made of rigid materials, for example, to maintain their shape and inner diameter size during use. In alternative embodiments, including the pyloric device 900 of Figs. 27A and 27B, a restrictor 908 may be pressure based or "pressure adjusting." In the pictured embodiment, the pyloric device 900 includes a stomach portion 902, a duodenal portion 904 and a pyloric portion 906, in addition to the pressure-based restrictor 908. The restrictor 908 is made of four leaflets that form a star-shaped opening, as well as a ring structure at the circular base of the four leaflets. Both the leaflets and the ring structure of the restrictor 908 can be designed to have a particular opening pressure threshold, to allow food to pass through once it reaches a certain pressure. When no food is pressing up against or passing through the restrictor 908, it may be partially open, as shown. When food in the end of the stomach begins to accumulate and press up against the restrictor 908, the leaflets will part at least somewhat, to increase the inner diameter of the restrictor 908 and begin to allow food to pass through the device 900. When food is no longer pressing against or passing through the restrictor 908, it will assume its default shape as illustrated. Thus, the restrictor is pressure based, in that it opens to a greater extent when the stomach is full and food exerts pressure on it, and it assumes a default, less open shape when food is not pressing against it.

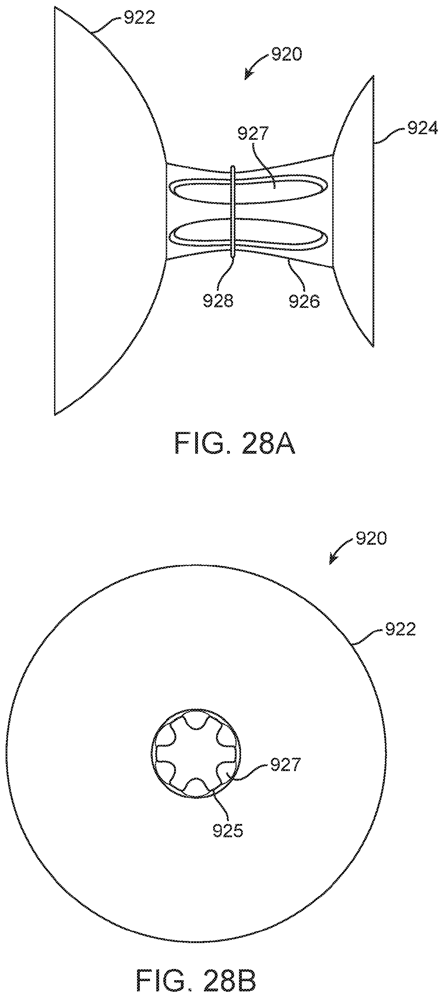

Referring now to Figs. 28A and 28B, another embodiment of a pyloric device 920 is illustrated in side view and front view, respectively. As with previous embodiments, this embodiment includes a stomach portion 922, a duodenal portion 924, and a pyloric portion 926. This pyloric device 920 is another example of an embodiment where the restriction feature is pressure based. Here, the pyloric portion 926 includes multiple longitudinal indentations 927 (or inward facing "ribs"), and a flexible band 928 (or "ring"), that is disposed around the outside of the pyloric portion. The band 928 acts as the restrictor in this embodiment. It may be made of rubber or other resilient material, and it is strong enough to constrict a section of the pyloric portion 926 that it encircles. As seen in Fig. 28B, the indentations 927 of the pyloric portion 926 form an inner surface 925 of the pyloric portion 926 that is shaped. The section with the band 928 is slightly constricted, but when food passes into the channel of the pyloric portion 926, the pressure of the food pressing out against the wall of the pyloric portion 926 is enough to stretch the band 928 outward at least slightly. Thus, similar to the previously described embodiment, the restrictor, which in this case is the band 928 working in conjunction with the indentations 927, is modulated by pressure of food substances moving through the pyloric portion 926.

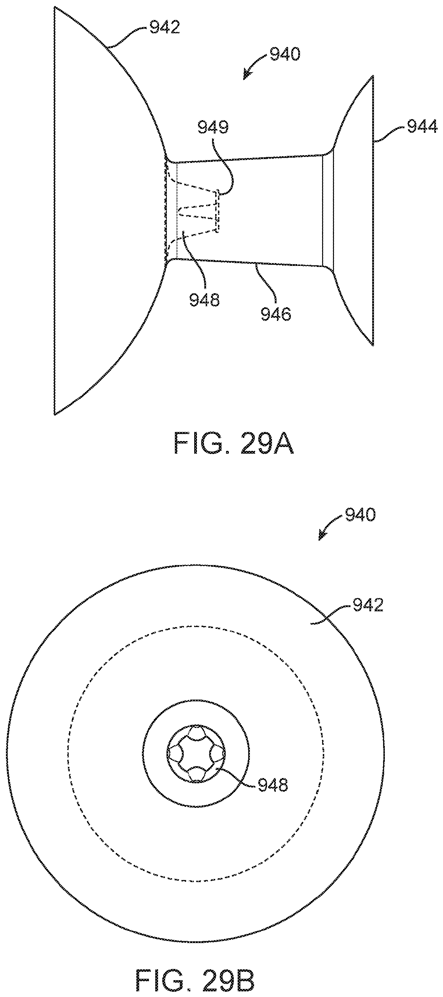

Figs. 29A and 29B show yet another embodiment of a pyloric device 940 that includes a pressure based restrictor 948. As with other embodiments, the pyloric device 940 includes a stomach portion 942, a duodenal portion 944 and a pyloric portion 946. The restrictor 948 has a partial cone shape, with small indentations, and at the small-diameter end, the restrictor 948 includes a ring 949. The ring 949 may be made of a resilient material, such as a shape memory metal or polymer, so that it may stretch and expand when food passes through the restrictor 948 and contract to its default shape when food is not passing through. Thus, again, the restrictor 948 is able to change shape when pressure from food is applied to it and resume a default shape when the pressure is removed.

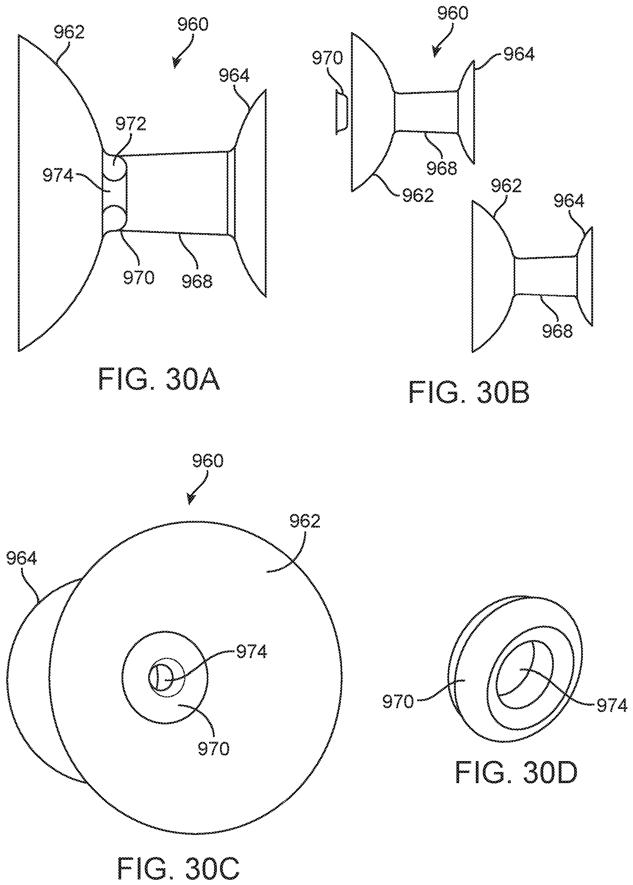

Referring now to Figs. 30A-30D, yet another embodiment of a pyloric device 960 is illustrated in cross section, side views, and perspective views, respectively. In this embodiment, the pyloric device 960 includes a stomach portion 962, a duodenal portion 964, a pyloric portion 968 and a restrictor 970. In this case, the restrictor 970 has a toroidal shape with an inner lumen 972 that may be filled with a gas or fluid. The restrictor 970 forms an opening 974, which has an inner diameter that is flexible and at least slightly expandable in response to the pressure of food pressing against it and/or passing through it. As shown in Fig. 30B, the restrictor 970 may be a separate piece attached between two layers of the pyloric device 960. The relative size of the opening 974 of the restrictor 970 is illustrated best in Fig. 30C. The restrictor 970 itself is pictured in detail in Fig. 30D. In various embodiments, the inflated restrictor 970 may be filled with any suitable substance and/or may have any suitable inner diameter and overall size.

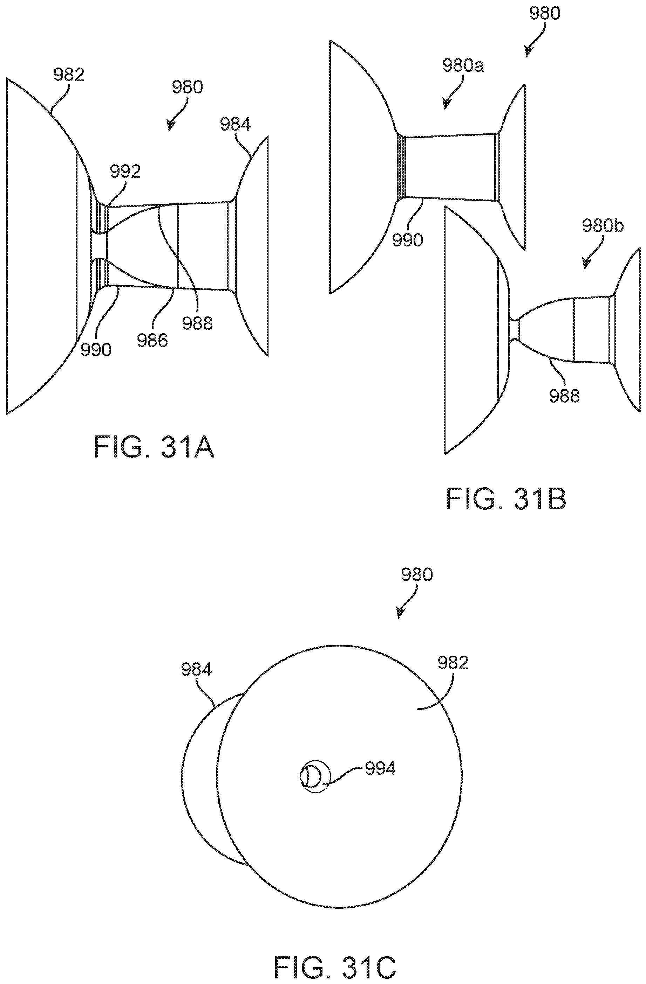

With reference now to Figs. 31A-31C, an alternative embodiment of a pyloric device 980 is illustrated. In this embodiment, the pyloric device 980 includes a stomach portion 982, a duodenal portion 984, and a pyloric portion 986, which includes an internal, circumferential space 992 that acts as a built-in restrictor. The space 992 is formed between an inner pyloric portion 988 and an outer pyloric portion 990. As illustrated in Fig. 31B, the pyloric device 980 may be formed by bringing together an outer layer 980a and an inner layer 980b. The inner pyloric portion 988 of the inner layer 980b is tapered (or "necked down") as it approaches the stomach portion 982. The outer pyloric portion 990 of the outer layer 980a has a shape that is closer to cylindrical. Thus, the two layers together form the space 992, that may be filled with air, water or other gases or fluids. Similar to the previous embodiment, the inner pyloric layer 988 may expand at least slightly in response from pressure of food pressing against and/or passing through it. Thus, the pyloric device 980 provides another example of a pressure based restrictor.

In any of the previously described embodiments of pyloric devices that include pressure-based restrictors, the restrictor portion may be located anywhere along the pyloric portion, from the stomach end to the duodenal end of the device. Furthermore, the restrictors may have any suitable inner diameters and may respond to any number of various ranges of pressure applied by food in the stomach. Additionally, any of these restrictors may be applied to other embodiments of pyloric devices described herein.

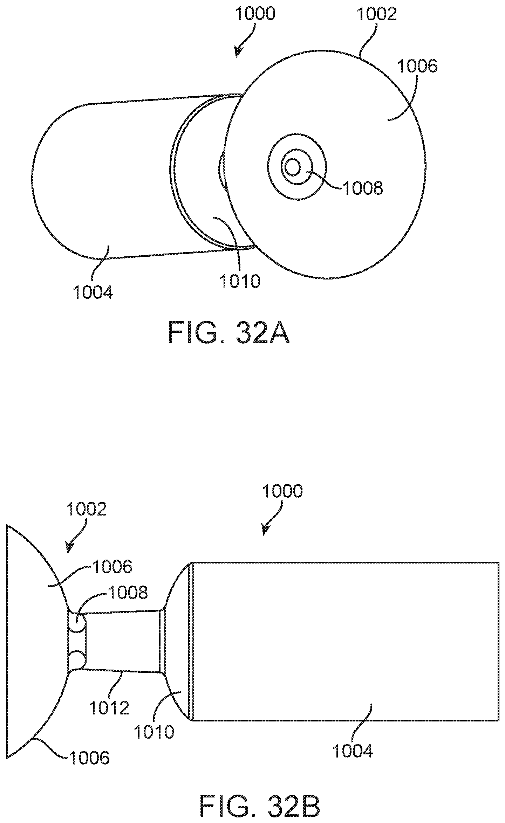

Figs. 32A and 32B show one embodiment of an obesity treatment system 1000, which includes a pyloric device 1002 and a duodenal sleeve 1004 attached to the pyloric device 1002. The sleeve 1004 may be used with any embodiment of the various pyloric devices described above. Here, the pyloric device 1002 is similar to that shown in Figs. 30A-30D and includes a stomach portion 1006, a duodenal portion 1010, a pyloric portion 1012 and an inflatable, toroidal restrictor 1008. The sleeve 1004 is attached to the duodenal portion 1010 of the pyloric device 1002 by any suitable method, such as adhesive or by making the sleeve and duodenal portion 1010 a one-piece structure. The sleeve may have any suitable length, thickness, material, etc.

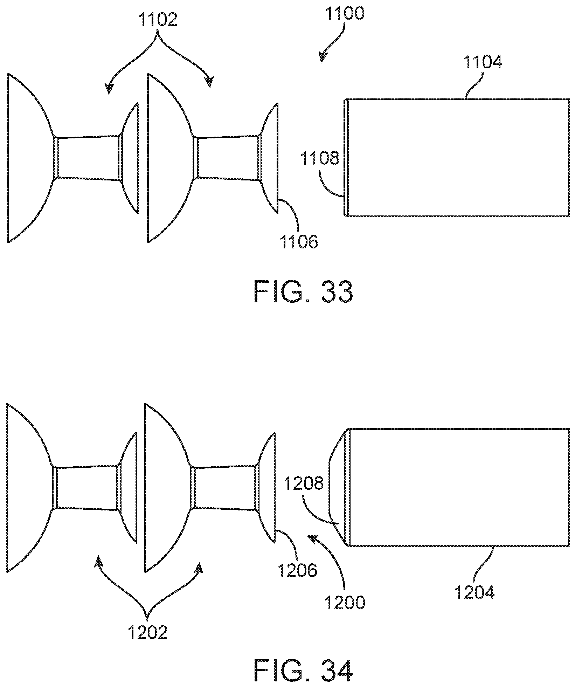

Fig. 33 illustrates one embodiment of an obesity treatment system 1100, with a two-layer pyloric device 1102 and an optional sleeve 1104. In this embodiment, the sleeve 1104 is cylindrical and has a proximal end 1108 that attaches to a distal end of the pyloric device 1102. The sleeve 1104 may optionally include longitudinal, helical or circumferential fibers in its wall for support.

Fig. 34 illustrates an alternative embodiment of an obesity treatment system 1200 including a pyloric device 1202 and an optional sleeve 1204. In this embodiment, the sleeve 1204 includes a proximal end with an opening. The duodenal portion 1206 of the pyloric device 1202 may be compressed and passed through the opening and then allowed to expand again to attach the pyloric device 1202 to the sleeve 1204.

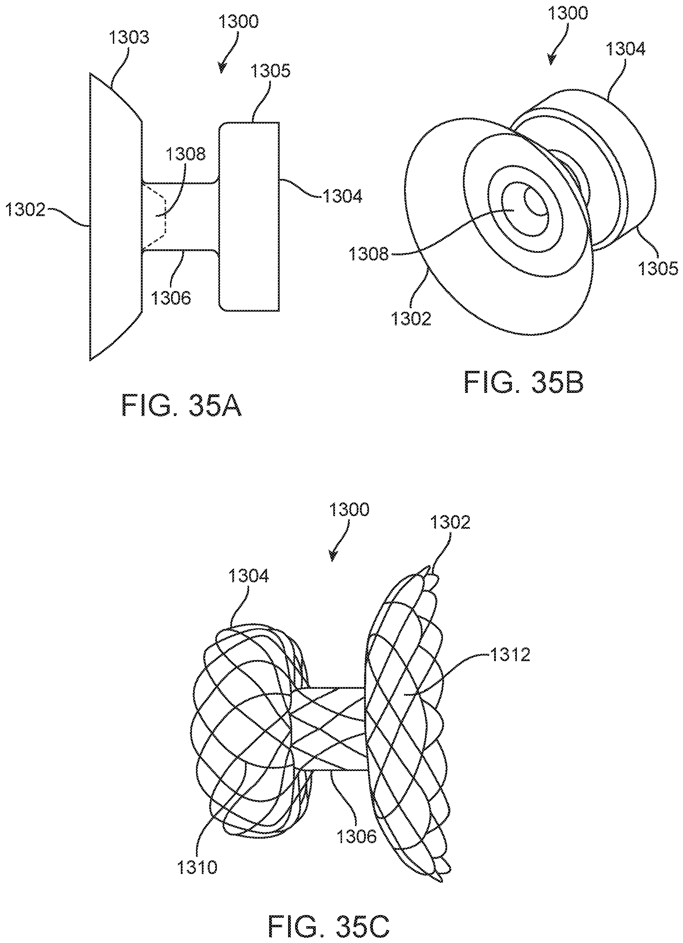

Figs. 35A and 35B are stylized drawings of an obesity treatment device 1300 having differently shaped proximal and distal portions, according to one embodiment. Fig. 35C is a side view of a braided, coated obesity treatment device 1300 having the general configuration of the device in Figs. 35A and 35B. As illustrated in Fig. 35A, the obesity treatment device 1300 includes a stomach portion 1302 (or "proximal portion") with a curved sidewall 1303, a duodenal portion 1304 (or "distal portion") with a straight sidewall 1305, and a pyloric portion 1306 spanning between the two. The device 1300 also includes a restrictor 1308 at the stomach portion, for slowing the passage of food out of the stomach. The restrictor 1308 may be integral with the stomach portion 1302 (i.e., one piece) or may be a separate, attached piece, according to alternative embodiments. Although the shapes of the curved sidewall 1303 and the straight sidewall 1305 may be advantageous for helping the device 1300 to fit well within the stomach and duodenum, in alternative embodiments, both sidewalls may be straight, both may be curved, or the sidewall of the proximal portion 1302 may be straight, and the sidewall of the distal portion 1304 may be curved. Figs. 35A and 35B are stylized drawings, and according to various embodiments, the obesity treatment device 1300 may be made of any suitable materials and combinations of materials, such as but not limited to the example illustrated in Fig. 35C.

Referring now to Fig. 35C, in some embodiments, the obesity treatment device 1300 may include a support member 1310 and a material 1312 (or "implant material") disposed over the support structure 1310. The support member 1310 may be a braided material and may be one piece or multiple pieces attached together. In one embodiment, for example, the braided material used to make the stomach portion 1302 and the duodenal portion 1304 may be different from the braided material used to make the pyloric portion 1306. For example, the material of the stomach portion 1302 and the duodenal 1304 portion may be a shape memory material, such as Nitinol or other shape memory metal, while the material of the pyloric portion 1306 may be a suture material, fabric or textile. (In other embodiments, the pyloric portion 1306 may have no structural support built into it but may instead simply be made the material 1312.) This combination may be advantageous, as it provides for the springing open of the stomach portion 1302 and the duodenal portion 1304 upon delivery out of a delivery device, while also providing a softer material for spanning the pylorus. Suture material, for example, provides structural support to the pyloric portion 1306, while allowing the pylorus to contract and work properly and without being so rigid as to potentially damage the pylorus.

Alternatively, the pyloric portion 1306 may be made of the same material as the stomach portion 1302 and the duodenal portion 1304, for example Nitinol or another shape memory material. In some embodiments, the support member 1310 may be one continuous piece of braided wire. In other embodiments, the pyloric portion 1306 may be made out of a different shape memory material than the stomach portion 1302 and/or the duodenal portion 1304. In any of this type of embodiment, the pyloric portion 1306 may open, upon delivery out of a delivery device, to a larger diameter than its delivery/constricted diameter.

In various embodiments, the implant material 1312 may be any suitable material or coating. In some embodiments, the material 1312 comes in two layers, and the support member 1310 is sandwiched between the two layers. In other embodiments, the material 1312 may be a coating applied to the outside and the inside of the support member 1310. The material 1312 may be any suitable polymer, such as but not limited to polytetrafluoroethylene PTFE, polyurethane or silicone, or any other suitable material.

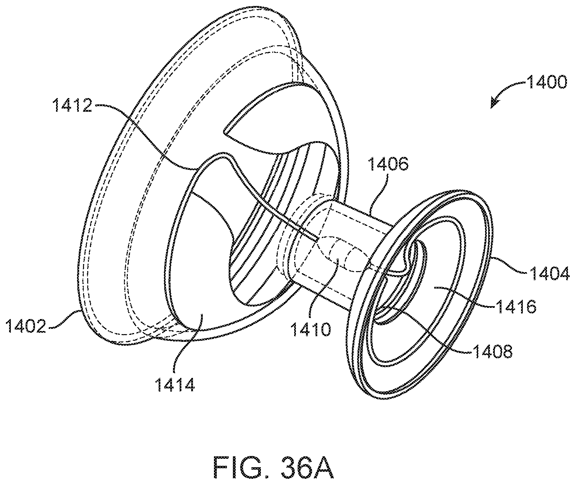

Figs. 36A-36C are side perspective, front perspective and rear perspective views, respectively, of an alternative embodiment of an obesity treatment device 1400 with a central restrictor 1410. As with previously described embodiments, the device 1400 includes a proximal portion 1402 for positioning in the stomach, a distal portion 1404 for positioning in the duodenum, and a pyloric portion 1406 for spanning the pylorus. In this embodiment, the pyloric portion 1406 includes a central lumen 1408 for passage of food, with a restrictor 1410 positioned in the middle of the lumen 1408 to slow food passage. In this embodiment, food passes around the restrictor as it passes through the lumen 1408. The restrictor 1410 is suspended in the lumen 1408 and held in place by a wire support member 1412, which is attached to the proximal portion 1402 via a proximal support member 1414 and to the distal portion 1404 via a distal support member 1416. The proximal support member 1414 and the distal support member 1416 are optional, and in an alternative embodiment the wire support member 1412 is included without the proximal or distal support members 1414, 1416. The restrictor 1410 may be made of any suitable material. In some embodiments, the restrictor 1410 may be expandable upon delivery, for example an inflatable balloon or shape memory member. In some embodiments, the wire support member 1416 and the restrictors 1410 may be removable after implantation and may be replaced with a larger or smaller sized support member/restrictor, for example to adjust a treatment. Alternatively, the restrictor 1410 may have a fixed shape and size. The wire support member 1412 may be made of any suitable material, such as but not limited to a metal or plastic.

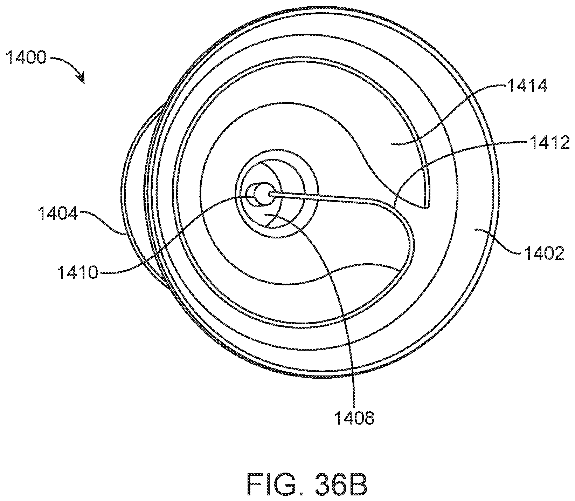

Fig. 36B shows the device 1400 from a front or proximal-to-distal view, as if looking from inside the stomach toward the duodenum. In this view, the restrictor 1410 can be seen "floating" within the lumen 1408 of the pyloric portion 1406. The wire support member 1412 can also be seen, attached to the proximal portion 1402 of the device 1400.

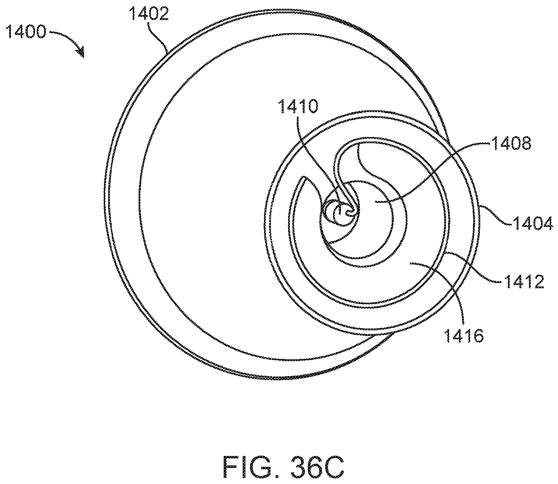

Fig. 36C shows the device 1400 from a rear or distal-to-proximal view, as if looking from inside the duodenum toward the stomach. The restrictor 1410 can again be seen floating within the lumen 1408 of the pyloric portion 1406. The wire support member 1412 can be seen, attached to the distal portion 1404 of the device 1400.

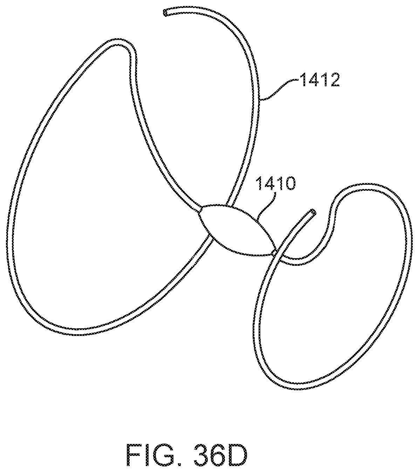

Fig. 36D shows the central restrictor 1410 and the wire support member 1412 by themselves. Again, in some embodiments, the wire support member 1412 is the only support for the central restrictor 1410-i.e., the device 1400 does not include a proximal or distal support member for the restrictor 1410.

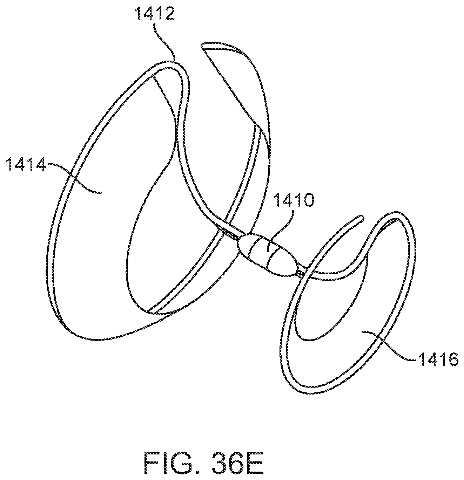

Fig. 36E shows the central restrictor 1410, the wire support member 1412 and the optional proximal support member 1414 and distal support member 1416. The proximal support member 1414 and the distal support member 1416 may help securely attach the wire support member 1412 to the proximal portion 1402 and the distal portion 1404 and thus provide additional support for the central restrictor 1410. In an alternative embodiment, the wire support member 1412 and the restrictor 1410 may be used as a stand-alone device, without any of the other surrounding structures just described.

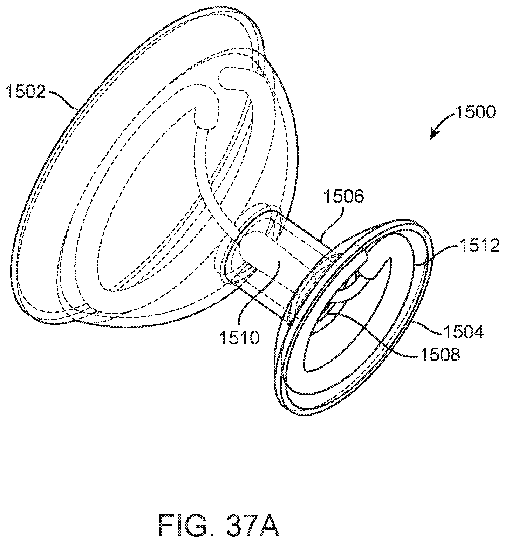

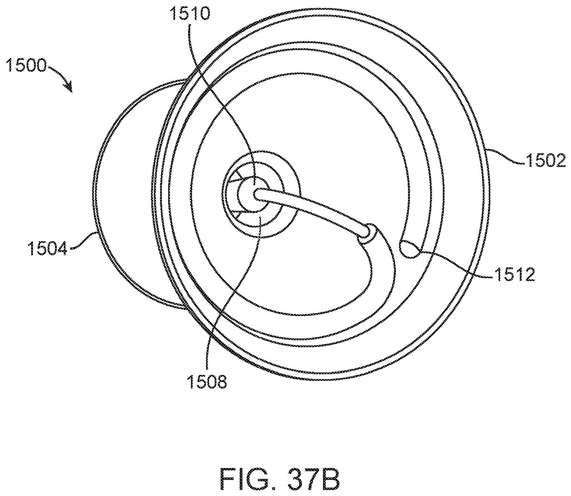

Figs. 37A and 37B are side perspective (partially transparent) and front perspective views, respectively, of another alternative embodiment of an obesity treatment device 1500. This embodiment includes a proximal portion 1502, a distal portion 1504, a pyloric portion 1506 with a lumen 1508, a central restrictor 1510, and a support member 1512. In this embodiment, the support member 1512 and the central restrictor may both be inflatable or expandable. Alternatively, either or both may have fixed sizes and shapes. As is apparent from the figures, the support member 1512 is wider than the wire support member 1412 of the previously described embodiment. As with the previously described embodiment, in some embodiments the support member 1512 and the restrictor 1510 may be used as a stand-alone device, and in some embodiments they may be removable and replaceable after implantation.

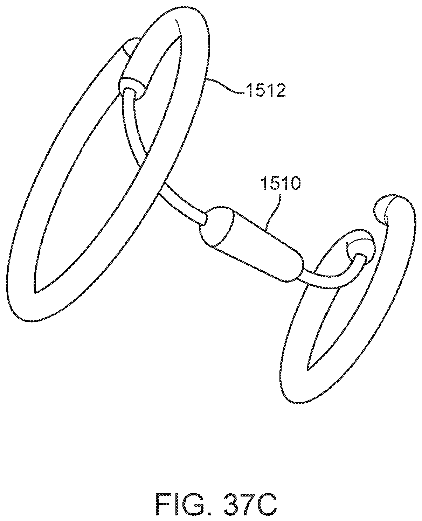

Fig. 37C shows the central restrictor 1510 and the support member 1512, apart from the device 1500.

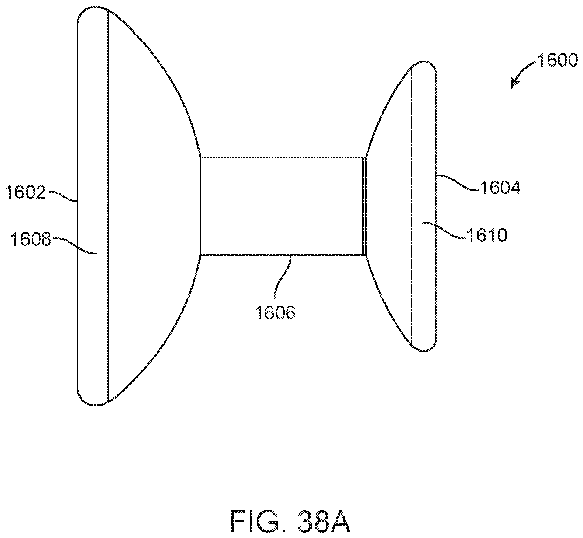

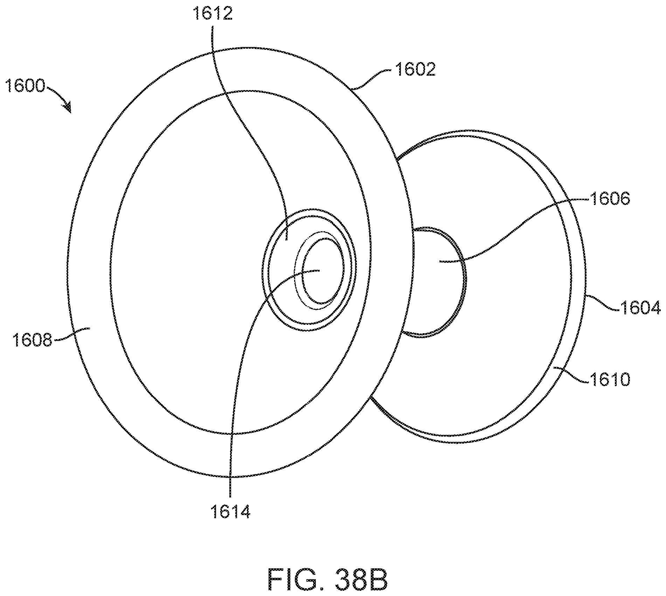

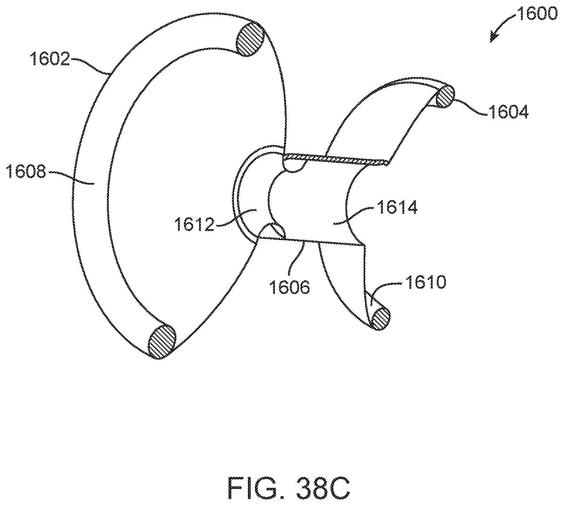

Figs. 38A-38C are side, perspective and cross-sectional views, respectively, of another alternative embodiment of an obesity treatment device 1600. As with many of the previously described embodiments, the device 1600 includes a proximal portion 1602 for placement in the stomach, a distal portion 1604 for placement in the duodenum, and a spanning pyloric portion 1606. To provide anchoring support, the device also includes two toroidal support members at opposite ends-a proximal support member 1608 and a distal support member 1610. As best seen in Figs. 38B and 38C, the pyloric portion 1606 forms an inner lumen 1614, and the device 1600 includes a restrictor 1612 at the proximal end of the lumen 1614 for slowing passage of food through the pylorus. As illustrated in Fig. 38C, the proximal support member 1608 and the distal support member 1610 are hollow. In some embodiments, the support members 1608, 1610 may be inflatable from a collapsed configuration for delivery to an inflated/expanded configuration after deployment. In alternative embodiments, the support members 1608, 1610 may be made of shape memory material and may not require inflation, thus simply springing open upon delivery out of a delivery device. In yet other embodiments, the support members 1608, 1610 may be prefilled with a substance that allows the support members 1608, 1610 to be collapsed down for delivery and then expand upon deployment. Other alternative embodiments may include only one toroidal support member at one end of the device 1600 and not at the other end. In some embodiments, the restrictor 1612 may also be hollow and may have any of the characteristics of the support members 1608, 1610 described here.

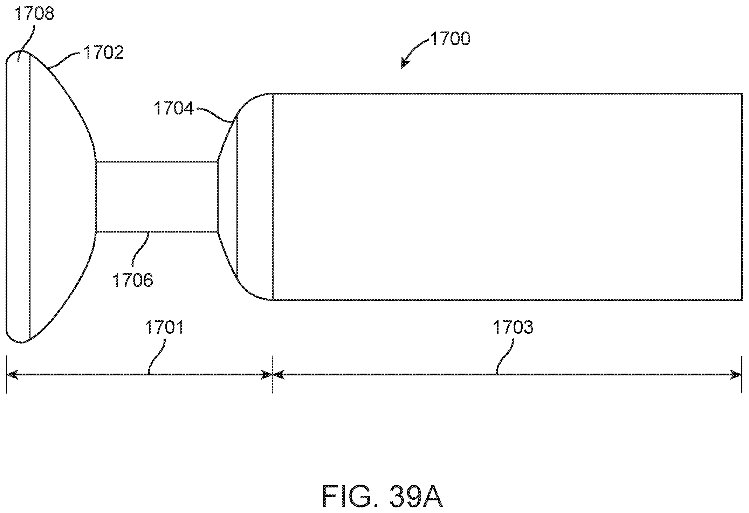

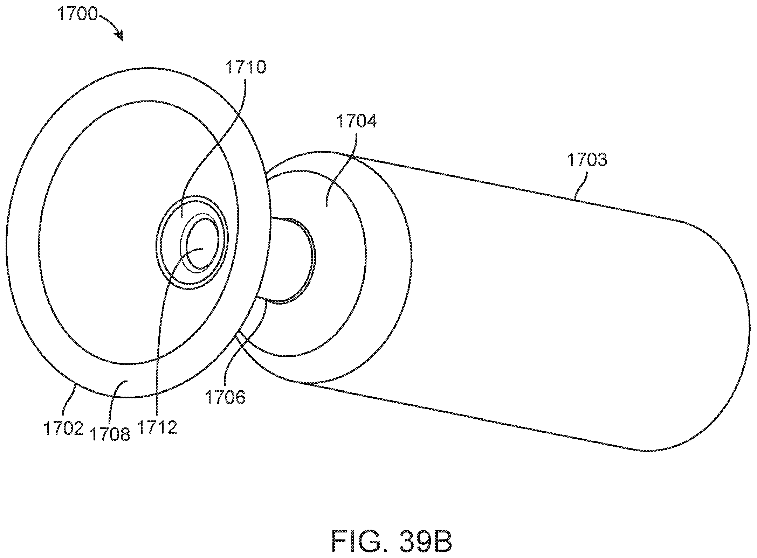

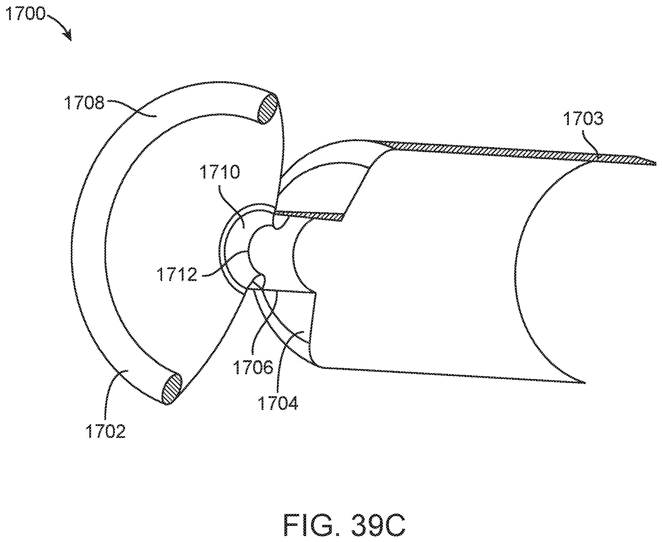

Referring now to Figs. 39A-39C, side, perspective and cross-sectional views, respectively, of an obesity treatment device 1700 according to another alternative embodiment are shown. In this embodiment, the device 1700 includes an anchoring portion 1701 and a duodenal sleeve 1703. The anchoring portion 1701 (or simply "anchor") includes a proximal portion 1702 with a toroidal support member 1708, a distal portion 1704, a pyloric portion 1706 that forms a lumen 1712, and a restrictor 1710 at the proximal end of the lumen 1712. The sleeve 1703 is attached to the distal portion 1704 and extends into the duodenum when the device 1700 is deployed. The support member 1708 and the restrictor may have any of the characteristics described above.

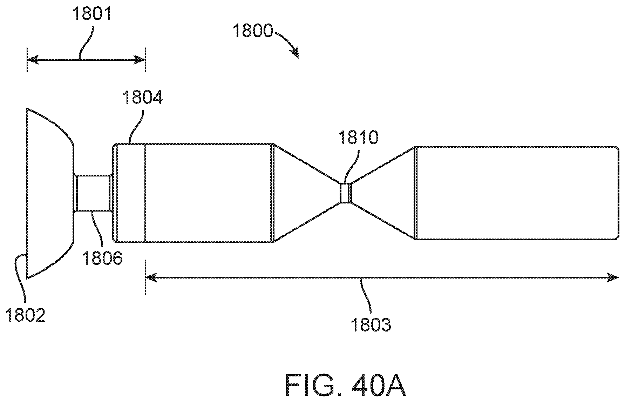

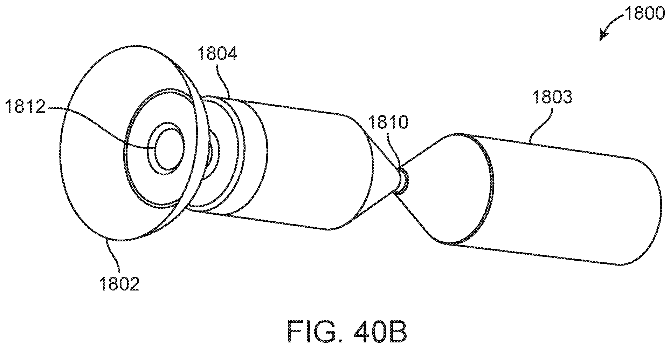

Referring to Figs. 40A and 40B, side and perspective views, respectively, of an obesity treatment device 1800 according to yet another embodiment are provided. Like the previous embodiment, this device 1800 includes an anchoring portion 1801 and a duodenal sleeve 1803. The anchoring portion includes a proximal portion 1802, a distal portion 1804, and a pyloric portion 1806 forming a lumen 1812. The anchoring portion 1801 does not include any food-slowing restrictor in this embodiment. Instead, the sleeve 1803 includes a restrictor 1810, which in this embodiment is formed as a narrowed portion of the sleeve 1803. The narrowed portion restrictor 1810 may be located anywhere along the length of the sleeve 1803, from its proximal end to its distal end. This device 1800 is but one example of a type of embodiment in which the restrictor 1810 is located in the sleeve 1803, rather than in the anchoring portion 1801. In alternative embodiments, any suitable type, shape, size and configuration of restrictor described above may be used in a duodenal sleeve, rather than in an anchoring portion.

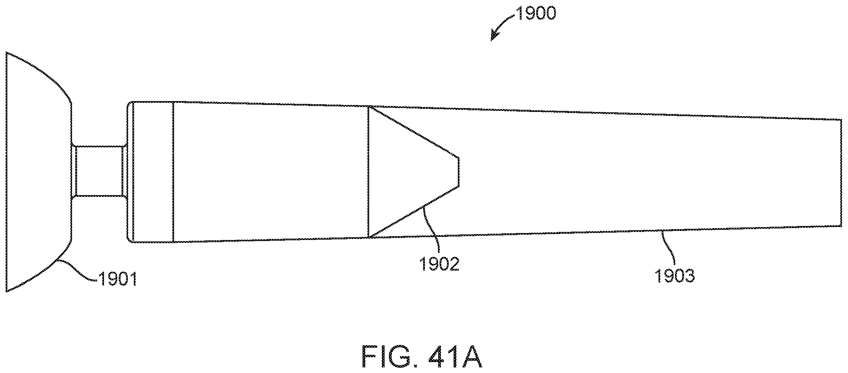

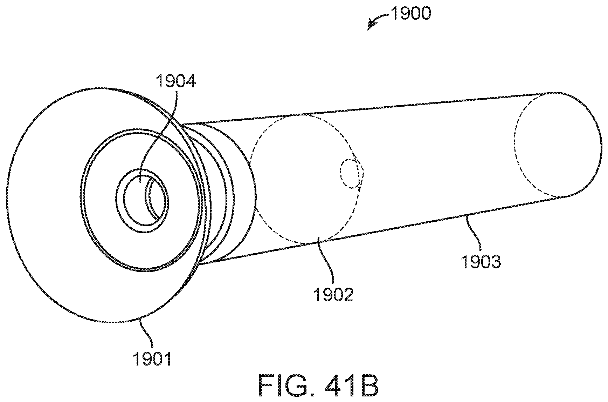

Figs. 41A and 41B are partially transparent side and perspective views, respectively, of an obesity treatment device 1900 according to yet another embodiment. In this embodiment, the device 1900 again includes an anchoring portion 1901 and a duodenal sleeve 1903. The food-slowing restrictor 1902 in this embodiment is a cone-shaped insert positioned within the sleeve 1902. The lumen 1904 formed by the pyloric portion 1906 does not include a restrictor-i.e., the only restrictor 1902 is in the sleeve 1903. Again, in various embodiments the insert restrictor 1902 may be positioned at any suitable location along the length of the sleeve 1903, from proximal end to distal end.

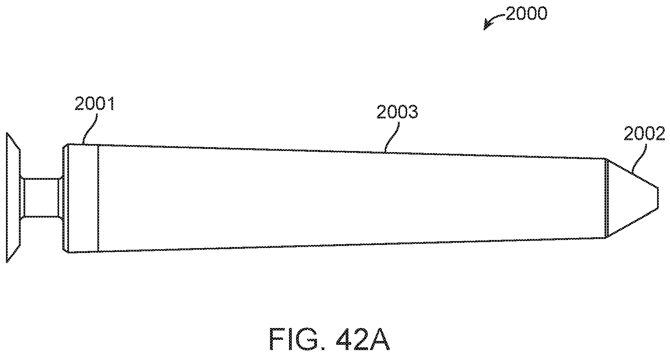

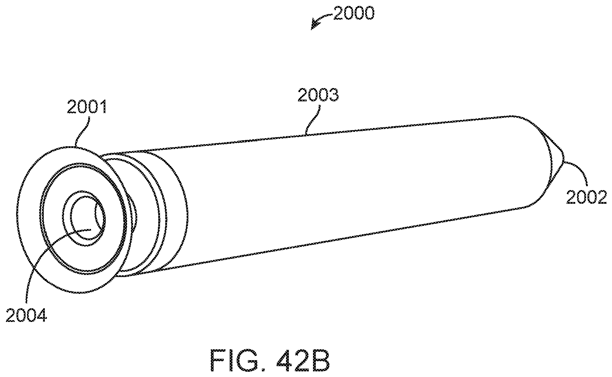

With reference now to Figs. 42A and 42B, side and perspective views, respectively, of another embodiment of an obesity treatment device 2000 are shown. This embodiment includes an anchoring portion 2001 and a duodenal sleeve 2003. In this embodiment, however, the device 2000 includes a cone-shaped restrictor 2002 attached to the extreme distal end of the sleeve 2003. Like the previously described embodiment, the lumen 2004 formed by the pyloric portion 2006 does not include a restrictor-i.e., the only restrictor 2002 is attached to the distal end of the sleeve 2003.

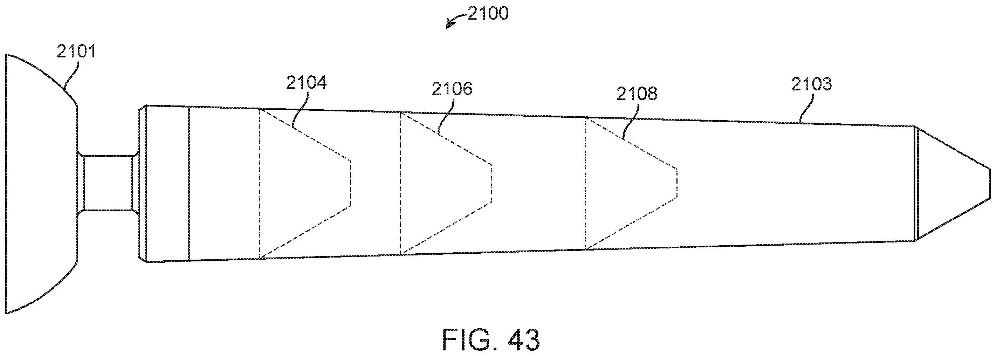

Referring to Fig. 43, another embodiment of an obesity treatment device 2100 is illustrated. As with some of the previous embodiments, the device 2100 includes an anchoring portion 2101 (or "pyloric device") and a sleeve 2103. This embodiment includes three, cone-shaped restrictors 2104, 2106, 2108, which are implanted within the lumen of the sleeve 2103. The restrictors 2104, 2106, 2108 may be positioned in any suitable location within the sleeve 2103, and they may be spaced apart from one another with the same or different spacing. For example, the first restrictor 2104 and the second restrictor 2106 are closer together than the second restrictor 2106 and the third restrictor 2108. Alternative embodiments may include two restrictors, four restrictors or any other number of restrictors.

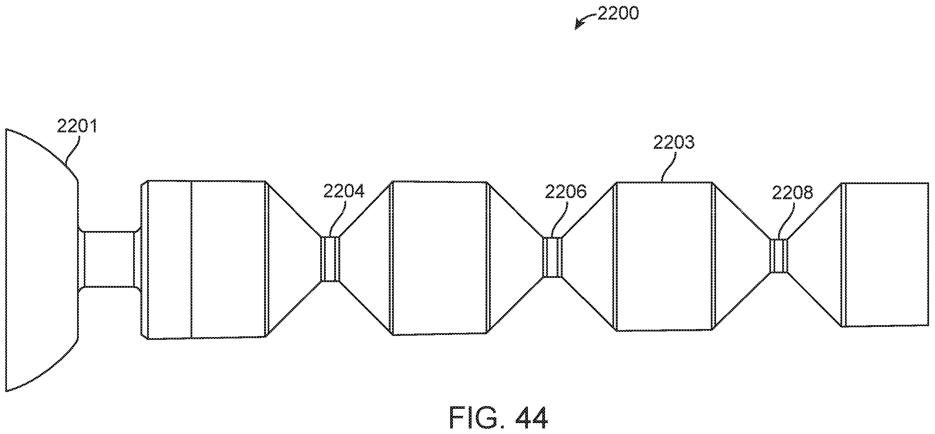

Referring to Fig. 44, another embodiment of an obesity treatment device 2200 is illustrated. The device 2200 includes an anchoring portion 2201 (or "pyloric device") and a sleeve 2203. This embodiment includes three restrictors 2204, 2206, 2208, which are formed as constrictions in (e.g., smaller diameter portions of) the sleeve 2203. The restrictors 2204, 2206, 2208 may be positioned at any suitable location along the length of the sleeve 2203, and they may be spaced apart from one another with the same or different spacing. In this example, the three restrictors 2204, 2206, 2208 are evenly spaced. Alternative embodiments may include two restrictors, four restrictors or any other number of restrictors.

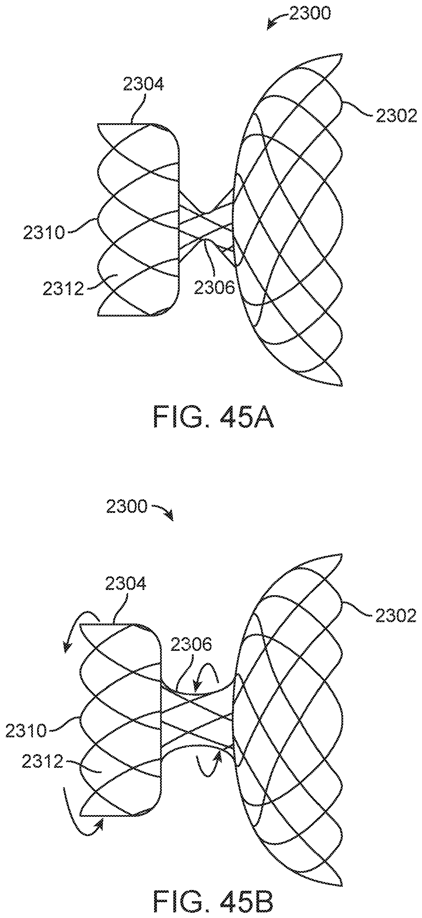

Figs. 45A-45C are side views of another embodiment of an obesity treatment device 2300. As with previous embodiments, this device 2300 includes a stomach portion 2302 for residing and anchoring the device in the stomach, a duodenal portion 2304 for placement in the duodenum, and a pyloric portion 2306 for spanning the pylorus between the two other portions 2302, 2304. In this embodiment, the pyloric portion 2306 is designed to change its shape from a twisted configuration, which slows passage of food out of the stomach and through the device 2300, to an untwisted configuration, which allows passage of food. Between the twisted, default configuration and the completely untwisted configuration, the pyloric portion 2306 may assume any incremental amount of openness-i.e., partially untwisted configurations.

As in some of the previously described embodiments, the obesity treatment device includes a support structure 2310 and an implant material 2312 attached to and surrounding the support structure 2310. In this embodiment, the support structure 2310 is formed as a braided, stent-like structure. The braided material used to make the support structure 2310 may be any shape memory material, such as but not limited to Nitinol or other shape memory metals. In this embodiment, the support structure 2310 is one piece of braided shape memory material. In alternative embodiments, the support structure 2310 may be made of multiple attached pieces of material, such as one piece of material for the stomach portion 2302, one piece of material for the duodenal portion 2304 and one piece of material for the pyloric portion 2306. The implant material 2312 may be any suitable coating or one or more pieces of material. For example, in some embodiments, the implant material 2312 may be two sheets of material that sandwich the support structure 2310 between them. Any suitable polymeric or other material may be used as the implant material 2312.

As illustrated in Fig. 45A, the obesity treatment device 2300 is provided with the pyloric portion 2306 in a default twisted configuration. In the twisted configuration, the pyloric portion 2306 has a relatively small diameter. Inside the device 2300 (not visible in Fig. 45A), the lumen of the pyloric portion 2306 in the twisted configuration may be completely closed or form a small channel, thus slowing or stopping the passage of food through the device 2300. The obesity treatment device 2300 will typically be delivered into the patient's stomach, pylorus and duodenum with the pyloric portion 2306 in the twisted configuration.

As illustrated in Fig. 45B, when a predetermined amount of pressure (hollowtipped, straight arrow) is applied to the stomach portion 2302 by food or chyme in the stomach, the pyloric portion 2306 begins to untwist into a more open configuration, which will allow the food or chyme to pass more quickly through the lumen of the pyloric portion 2306. In this configuration, however, the pyloric portion 2306 still slows passage of food through the device 2300, but not as much as passage is slowed in the twisted, default configuration of Fig. 45A. Untwisting of the pyloric portion 2306 also results in rotation (solid-tipped, curved arrows) of the duodenal portion 2304 about the central axis of the device 2300. Rotation of the duodenal portion 2304 and/or the pyloric portion 2306 may be facilitated by providing a coating or surface material on the duodenal portion 2304.

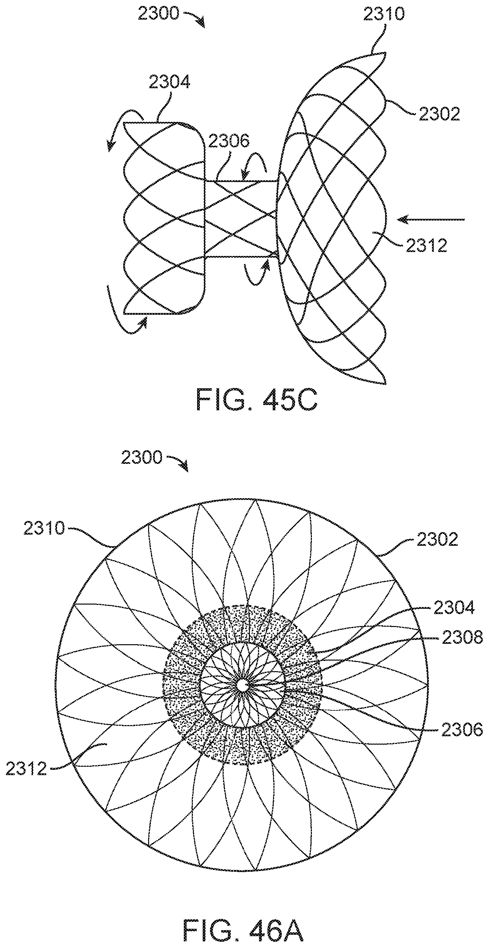

Fig. 45C illustrates the obesity treatment device 2300 in a fully untwisted configuration, in which food will pass yet more quickly through the pyloric portion 2306 and into the duodenum. According to various embodiments, the pyloric portion 2306 of the obesity treatment device 2306 may be designed to begin untwisting at a specific pressure on the stomach portion 2302 and may become fully untwisted at another specific pressure, each of such specific pressures being within any suitable range. For example, the pyloric portion 2306 may start to untwist when a pressure of between about 0.0267 bar (20 mmHg) and about 0.0533 bar (40 mmHg) is applied to the stomach portion 2306 by food. The pyloric portion 2306 may become fully untwisted at a pressure of between about 0.1067 bar (80 mmHg) and about 0.1333 bar (100 mmHg), in some embodiments. These ranges are only exemplary, however, and alternative embodiments of the obesity treatment device 2300 may untwist at pressures outside these ranges.

After food has passed through the device 2300 and pressure on the stomach portion 2302 has been relieved, the pyloric portion 2306 will twist back into its twisted, default configuration. Depending on the amount of pressure applied to the stomach portion 2302 at any given time, the pyloric portion 2306 may open partway or all the way. Thus, there is a gradation of opening and closing of the pyloric portion 2306, which provides for incremental slowing of gastric emptying without completely blocking gastric emptying.

Figs. 46A-46C show the same obesity treatment device 2300 of Figs. 45A-45C, but from a front view, as if looking from the stomach through the device 2300 into the duodenum. Fig. 46A shows the obesity treatment device 2300 in the twisted, default configuration, with the pyloric portion 2306 forming a channel 2308 (or "lumen" or "passage") to slow the passage of food through the device 2300. In some embodiments, the channel 2308 of the pyloric portion 2306 may be completely closed in the twisted, default configuration. In other embodiments, however, the channel 2308 is partially open in the twisted configuration. For example, the channel 2308 when the pyloric portion 2306 is twisted may have an inner diameter of about 3 mm to about 8 mm, in some embodiments. Inner diameters smaller than 3 mm or greater than 8 mm are also possible, however, in alternative embodiments.

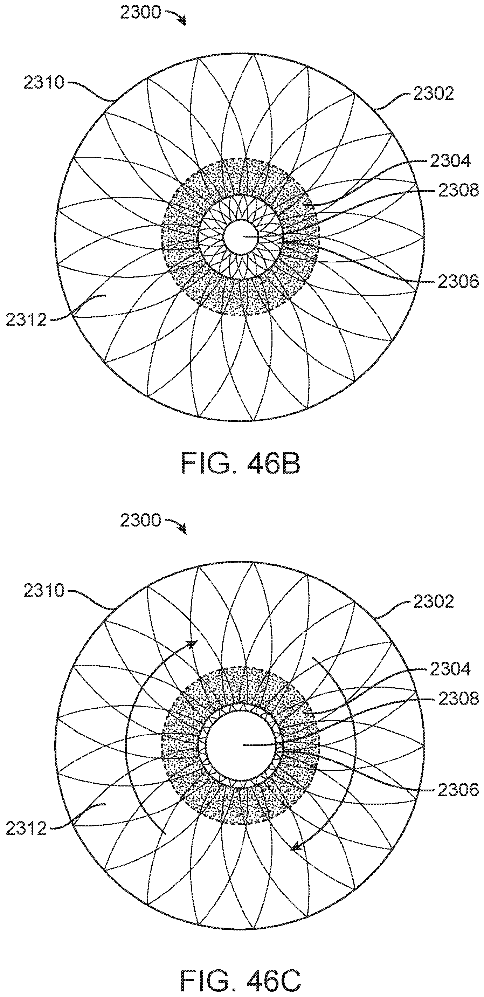

Fig. 46B shows the device 2300 with the pyloric portion 2306 in the partially untwisted configuration, with the channel 2308 having a wider diameter than in Fig. 46A. The solid-tipped, curved arrows illustrate the rotation of the duodenal portion 2304 around the central axis of the device 2300, in response to the untwisting/rotating of the pyloric portion 2306 about the same central axis. Fig. 46C shows the obesity treatment device 2300 with the pyloric portion 2306 in the untwisted configuration. In various embodiments, the channel 2308 may have any suitable diameters in its untwisted configuration. For example, the channel 2308 may have a maximum internal diameter, when the pyloric portion 2306 is untwisted, of between about 9 mm and about 15 mm. In alternative embodiments, smaller or larger diameters are possible. All suitable combinations are contemplated within the scope of the present application.

In some embodiments, the obesity treatment device 2300 may be adjustable by a user. For example, such adjustment(s) may be made before the device 2300 is implanted in the patient. One such adjustment, or set of adjustments, may be of the diameter of the channel 2308. For example, the user might be able to adjust the pyloric portion 2306 such that the channel 2308 has a desired inner diameter in the twisted, default configuration. Alternatively or additionally, the pyloric portion 2306 might be adjustable such that the channel 2308 has a desired maximum inner diameter in the untwisted configuration. Other possible adjustments, in some embodiments, might include the specific pressure at which the pyloric portion 2306 starts to untwist and/or the specific pressure at which the pyloric portion 2306 assumes the untwisted configuration of Figs. 45C and 46C. Any or all of these adjustments by the user might be possible, according to various embodiments.

In summary, the obesity treatment device 2300 of Figs. 45A-45C and 46A-46C adjusts itself to allow passage of food out of the stomach, in response to the presence of food in the stomach, which applies pressure against the stomach portion 2302 of the device 2300. The pyloric portion 2306 moves from twisted to untwisted and back to twisted, with any number of partially twisted configurations along the way, to facilitate slowing of the passage of food while still allowing food to pass. Thus, the device 2300 provides for "real time," automatic adjustment, based on the amount and/or presence of food in the stomach.

As mentioned several times above, whenever suitable, any of the features of any embodiments described in this application may be combined with any other embodiments to provide an alternative embodiment. For example, any restrictor described above in relation to one embodiment may be used in a different embodiment. Similarly, any duodenal sleeve described above may be combined with any anchoring portion described above. For the anchoring portions of the obesity devices described herein, different shapes, sizes and types of stomach (or "proximal") portions, pyloric (or "middle") portions and duodenal (or "distal") portions may be used in different combinations, as practicable. Support members within a pyloric device may be combined in different ways. Restrictors may be located anywhere along the length of the pyloric portion or along the length of a duodenal sleeve, according to various embodiments. In short, any combination may be made of any of the features described above, without departing from the scope of the invention.

In some embodiments, any of the pyloric devices described above may be designed and/or fabricated using a customized method, to provide a device specifically designed for a given patient. In general, such custom method may involve using one or more images of the patient's pyloric region to 3D print or otherwise fabricate the pyloric device. One embodiment, for example, starts by acquiring or receiving one or more CT or MRI images of the patient's GI tract, in the area of the end of the stomach, the pylorus and the beginning of the duodenum. The physician (and/or a computer program configured to automatically design a pyloric device) may then select one or more aspects of the pyloric implant for the particular patient, for example the size of a restrictor for the device, and whether the device will include a sleeve, etc. The physician may then send the patient's CT/MRI images and plan to a manufacturer, which may segment the CT/MRI images to extract relevant anatomical information for making the pyloric device and to design and size the device, based on the patient's anatomy and the physician's plan. The design may then be sent to a 3D printer, which may then make the implant. Finally, the pyloric device is sent back to the physician, who will use it on the patient for obesity treatment. This is merely one exemplary method, and alternative embodiments may include different steps and/or differently ordered steps.

## Claims

1. A device (10, 120, 130, 140, 150, 160, 200, 300, 310, 320, 400, 430, 500, 530, 540, 550, 560, 570, 600, 700, 800, 900, 920, 940, 960, 980, 1002, 1102, 1202, 1300, 1400, 1500, 1600, 1700, 1900 2100, 2300) for implantation in a pylorus between a stomach and duodenum for promoting weight loss, the device comprising:
a stomach portion (18, 112, 132, 142, 202, 432, 502, 552, 562, 602, 572, 702, 902, 922, 942, 962, 982, 1006, 1302, 1402, 1502, 1602, 2302) configured to expand from a collapsed configuration to a first maximum diameter to anchor the device in the stomach;
a pyloric portion (124, 114, 134, 144, 204, 434, 504, 554, 564, 604, 574, 704, 904, 926, 946, 968, 984, 986, 1012, 1306, 1406, 1506, 1606, 1806, 2306) extending from the stomach portion and having a second maximum diameter;
a duodenal portion (20, 116, 146, 136, 206, 436, 506, 556, 610, 576, 706, 906, 924, 944, 964, 1010, 1206, 1304, 1404, 1504, 1604, 2304) extending from the pyloric portion and configured to expand from a collapsed configuration to a third maximum diameter;
a channel (84, 129, 139, 205, 555, 710, 1408, 1508, 1614, 2308) extending through the stomach portion, the pyloric portion and the duodenal portion, for allowing passage of food material through the device from the stomach to the duodenum,
wherein each of the stomach portion, the pyloric portion and the duodenal portion comprises:
a support member (320, 322, 324, 326, 328, 330, 408, 410, 438, 440, 503, 533, 558, 578, 1310, 1412, 1512, 1608, 1610, 2310); and
an implant material attached to the support member,
wherein the first maximum diameter and the third maximum diameter are both larger than the second maximum diameter, and
wherein at least one feature of the device is configured to slow the passage of the food material through the channel; wherein the device comprises a restrictor (138, 720, 908, 924, 948, 970, 1008, 1308, 1410, 1510, 1612,) attached to the support member at or near the pyloric portion, wherein the at least one feature of the device configured to slow the passage of the food material through the channel comprises the restrictor,
**characterised**
**in that** the restrictor is located at a junction between the stomach portion and the pyloric portion and
**in that** the restrictor is expandable.

2. The device of claim 1, wherein the first maximum diameter is larger than the third maximum diameter.

3. The device of claim 1, wherein an inner diameter of the channel increases between a proximal opening of the pyloric portion at the stomach portion and a distal opening of the pyloric portion at the duodenal portion.

4. The device of claim 1, wherein an inner diameter of the channel decreases between a proximal opening of the pyloric portion at the stomach portion and a distal opening of the pyloric portion at the duodenal portion.

5. The device of claim 1, wherein the support member of the stomach portion, the pyloric portion and the duodenal portion comprises a single shape memory support stent that extends from a proximal end of the stomach portion, through the pyloric portion, to a distal end of the duodenal portion.

6. The device of claim 1, wherein the support member comprises three attached pieces of shape memory stent material, one for each of the stomach portion, the pyloric portion and the duodenal portion.

7. The device of claim 1, wherein the support member of the stomach portion and the support member of the duodenal portion each comprises a shape memory material, and wherein the support member of the pyloric portion comprises a different material attached at each end to the support member of the stomach portion and the support member of the duodenal portion, wherein the different material comprises a compliant material, and wherein the shape memory material is selected from the group consisting of Nitinol and other shape memory metals.

8. The device of claim 1, wherein the implant material comprises two layers of material, and wherein the support member is disposed between the two layers.

9. The device of claim 1, further comprising a coating applied to at least one of an inner surface or an outer surface of the device.

10. The device of claim 1, wherein the support member is selected from the group consisting of rings, star shaped members, a continuous wire extending from the stomach portion to the duodenal portion, and braided wire.

11. The device of claim 1, further comprising a restrictor built into the support member at or near the pyloric portion, wherein the at least one feature of the device configured to slow the passage of the food material through the channel comprises the restrictor.

12. The device of claim 1, further comprising a duodenal sleeve (78, 1004, 1204, 1703, 1803, 1903, 2003, 2103, 2203) attached to a distal end of the duodenal portion.

13. The device of claim 12, further comprising a restrictor (1902, 2002, 2104, 2106, 2108, 2204, 2206, 2208) coupled with or formed in the duodenal sleeve to slow the passage of food material through the duodenal sleeve.

14. The device of claim 1, wherein the pyloric portion is configured to change from a default, twisted configuration to an open, untwisted configuration, in response to a predefined amount of pressure applied to the stomach portion by food in the stomach, wherein the predefined amount of pressure comprises a range of pressures comprising a smallest amount of pressure at which the pyloric portion starts to untwist and a largest amount of pressure, which is required for the pyloric portion to completely untwist and wherein the smallest amount of pressure is between 0.0267 bar (20 mmHg) and 0.0533 bar (40 mmHg), and wherein the largest amount of pressure is at least 0.1067 bar (80 mmHg).

## Patentansprüche

1. Vorrichtung (10, 120, 130, 140, 150, 160, 200, 300, 310, 320, 400, 430, 500, 530, 540, 550, 560, 570, 600, 700, 800, 900, 920, 940, 960, 980, 1002, 1102, 1202, 1300, 1400, 1500, 1600, 1700, 1900, 2100, 2300) zur Implantation in einen Pylorus zwischen einem Magen und einem Duodenum zur Förderung von Gewichtsverlust, die Vorrichtung umfassend:
einen Magenabschnitt (18, 112, 132, 142, 202, 432, 502, 552, 562, 602, 572, 702, 902, 922, 942, 962, 982, 1006, 1302, 1402, 1502, 1602, 2302), der konfiguriert ist, um sich von einer faltbaren Konfiguration auf einen ersten maximalen Durchmesser zu erweitern, um die Vorrichtung im Magen zu verankern;
einen Pylorusabschnitt (124, 114, 134, 144, 204, 434, 504, 554, 564, 604, 574, 704, 904, 926, 946, 968, 984, 986, 1012, 1306, 1406, 1506, 1606, 1806, 2306), der sich von dem Magenabschnitt erstreckt und einen zweiten maximalen Durchmesser aufweist;
einen Duodenalabschnitt (20, 116, 146, 136, 206, 436, 506, 556, 610, 576, 706, 906, 924, 944, 964, 1010, 1206, 1304, 1404, 1504, 1604, 2304), der sich vom Pylorusabschnitt aus erstreckt und konfiguriert ist, um sich von einer faltbaren Konfiguration auf einen dritten maximalen Durchmesser zu erweitern;
einen Kanal (84, 129, 139, 205, 555, 710, 1408, 1508, 1614, 2308), der sich durch den Magenabschnitt, den Pylorusabschnitt und den Duodenalabschnitt erstreckt, um den Durchgang von Nahrungsmittelmaterial durch die Vorrichtung vom Magen zum Duodenum zu ermöglichen,
wobei jeder der Magenabschnitte, der Pylorusabschnitte und der Duodenalabschnitte Folgendes umfasst:
ein Stützelement (320, 322, 324, 326, 328, 330, 408, 410, 438, 440, 503, 533, 558, 578, 1310, 1412, 1512, 1608, 1610, 2310); und
ein Implantatmaterial, das an dem Stützelement befestigt ist,
wobei der erste maximale Durchmesser und der dritte maximale Durchmesser beide größer als der zweite maximale Durchmesser sind, und
wobei mindestens ein Merkmal der Vorrichtung konfiguriert ist, um den Durchgang des Lebensmittelmaterials durch den Kanal zu verlangsamen;
wobei die Vorrichtung einen Begrenzer (138, 720, 908, 924, 948, 970, 1008, 1308, 1410, 1510, 1612) umfasst, der an dem Stützelement am oder in der Nähe des Pylorusabschnitts angebracht ist,
wobei das mindestens eine Merkmal der Vorrichtung, das konfiguriert ist, um den Durchgang des Lebensmittelmaterials durch den Kanal zu verlangsamen, den Begrenzer umfasst,
**dadurch gekennzeichnet, dass** der Begrenzer an einer Verbindungsstelle zwischen dem Magenabschnitt und dem Pylorusabschnitt angeordnet ist und dass der Begrenzer ausdehnbar ist.

2. Vorrichtung nach Anspruch 1, wobei der erste maximale Durchmesser größer ist als der dritte maximale Durchmesser.

3. Vorrichtung nach Anspruch 1, wobei ein Innendurchmesser des Kanals zwischen einer proximalen Öffnung des Pylorusabschnitts am Magenabschnitt und einer distalen Öffnung des Pylorusabschnitts am Duodenalabschnitt zunimmt.

4. Vorrichtung nach Anspruch 1, wobei ein Innendurchmesser des Kanals zwischen einer proximalen Öffnung des Pylorusabschnitts am Magenabschnitt und einer distalen Öffnung des Pylorusabschnitts am Duodenalabschnitt abnimmt.

5. Vorrichtung nach Anspruch 1, wobei das Stützelement des Magenabschnitts, des Pylorusabschnitts und des Duodenalabschnitts einen einzelnen Formgedächtnisstent umfasst, der sich von einem proximalen Ende des Magenabschnitts durch den Pylorusabschnitt zu einem distalen Ende des Duodenalabschnitts erstreckt.

6. Vorrichtung nach Anspruch 1, wobei das Stützelement drei angebrachte Stücke aus Formgedächtnisstentmaterial umfasst, jeweils eines für den Magenabschnitt, den Pylorusabschnitt und den Duodenalabschnitt.

7. Vorrichtung nach Anspruch 1, wobei das Stützelement des Magenabschnitts und das Stützelement des Duodenalabschnitts jeweils ein Formgedächtnismaterial umfassen, und wobei das Stützelement des Pylorusabschnitts ein anderes Material umfasst, das an jedem Ende mit dem Stützelement des Magenabschnitts und dem Stützelement des Duodenalabschnitts verbunden ist, wobei das andere Material ein nachgiebiges Material umfasst, und wobei das Formgedächtnismaterial aus der Gruppe ausgewählt ist, die aus Nitinol und anderen Formgedächtnismetallen besteht.

8. Vorrichtung nach Anspruch 1, wobei das Implantatmaterial zwei Materialschichten umfasst und das Stützelement zwischen den beiden Schichten angeordnet ist.

9. Vorrichtung nach Anspruch 1, ferner umfassend eine Beschichtung, die auf mindestens eine innere Oberfläche oder eine äußere Oberfläche der Vorrichtung aufgebracht ist.

10. Vorrichtung nach Anspruch 1, wobei das Stützelement ausgewählt ist aus der Gruppe, bestehend aus Ringen, sternförmigen Elementen, einem durchgehenden Draht, der sich vom Magenabschnitt zum Duodenalabschnitt erstreckt, und geflochtenem Draht.

11. Vorrichtung nach Anspruch 1, ferner umfassend einen Begrenzer, der in das Stützelement am oder in der Nähe des Pylorusabschnitts eingebaut ist, wobei das mindestens eine Merkmal der Vorrichtung, das zur Verlangsamung des Durchgangs des Nahrungsmittels durch den Kanal konfiguriert ist, den Begrenzer umfasst.

12. Vorrichtung nach Anspruch 1, ferner umfassend eine Duodenalhülse (78, 1004, 1204, 1703, 1803, 1903, 2003, 2103, 2203), die an einem distalen Ende des Duodenalabschnitts angebracht ist.

13. Vorrichtung nach Anspruch 12, ferner umfassend einen Begrenzer (1902, 2002, 2104, 2106, 2108, 2204, 2206, 2208), der mit der Duodenalhülse gekoppelt oder in ihr ausgebildet ist, um den Durchgang von Nahrungsmittelmaterial durch die Duodenalhülse zu verlangsamen.

14. Vorrichtung nach Anspruch 1, wobei der Pylorusabschnitt konfiguriert ist, um von einer standardmäßigen, verdrehten Konfiguration in eine offene, unverdrehte Konfiguration zu wechseln, als Reaktion auf einen vordefinierten Druckbetrag, der auf den Magenabschnitt durch die Nahrung im Magen ausgeübt wird, wobei der vordefinierte Druckbetrag einen Druckbereich umfasst, der einen kleinsten Druckbetrag, bei dem der Pylorusabschnitt beginnt, sich zu entdrehen, und einen größten Druckbetrag umfasst, der erforderlich ist, damit sich der Pylorusabschnitt vollständig entdreht, und wobei der kleinste Druckbetrag zwischen 0,0267 bar (20 mmHg) und 0,0533 bar (40 mmHg) liegt und wobei der größte Druckbetrag mindestens 0,1067 bar (80 mmHg) beträgt.

## Revendications

1. Dispositif (10, 120, 130, 140, 150, 160, 200, 300, 310, 320, 400, 430, 500, 530, 540, 550, 560, 570, 600, 700, 800, 900, 920, 940, 960, 980, 1002, 1102, 1202, 1300, 1400, 1500, 1600, 1700, 1900, 2100, 2300) destiné à être implanté dans un pylore entre l'estomac et le duodénum, pour favoriser la perte de poids, le dispositif comprenant :
une portion d'estomac (18, 112, 132, 142, 202, 432, 502, 552, 562, 602, 572, 702, 902, 922, 942, 962, 982, 1006, 1302, 1402, 1502, 1602, 2302) configurée pour s'étendre d'une configuration repliée à un premier diamètre maximal pour ancrer le dispositif dans l'estomac ;
une portion pylorique (124, 114, 134, 144, 204, 434, 504, 554, 564, 604, 574, 704, 904, 926, 946, 968, 984, 986, 1012, 1306, 1406, 1506, 1606, 1806, 2306) s'étendant de la portion gastrique et ayant un deuxième diamètre maximal ;
une portion duodénale (20, 116, 146, 136, 206, 436, 506, 556, 610, 576, 706, 906, 924, 944, 964, 1010, 1206, 1304, 1404, 1504, 1604, 2304) s'étendant de la portion pylorique et configurée pour s'étendre d'une configuration repliée à un troisième diamètre maximal ;
un canal (84, 129, 139, 205, 555, 710, 1408, 1508, 1614, 2308) s'étendant à travers la portion gastrique, la portion pylorique et la portion duodénale, pour permettre le passage du produit alimentaire à travers le dispositif de l'estomac au duodénum,
dans lequel chacune des portions gastrique, pylorique et duodénale comprend :
un élément de support (320, 322, 324, 326, 328, 330, 408, 410, 438, 440, 503, 533, 558, 578, 1310, 1412, 1512, 1608, 1610, 2310) ; et
un matériau d'implant fixé à l'élément de support,
dans lequel le premier diamètre maximal et le troisième diamètre maximal sont tous deux supérieurs au deuxième diamètre maximal, et
dans lequel au moins une caractéristique du dispositif est configurée pour ralentir le passage du produit alimentaire à travers le canal ;
dans lequel le dispositif comprend un restricteur (138, 720, 908, 924, 948, 970, 1008, 1308, 1410, 1510, 1612) fixé à l'élément de support au niveau ou à proximité de la portion pylorique,
dans lequel l'au moins une caractéristique du dispositif configurée pour ralentir le passage du produit alimentaire à travers le canal comprend le restricteur,
**caractérisé en ce que** le restricteur est situé à la jonction entre la portion gastrique et la portion pylorique et **en ce que** le restricteur est extensible.

2. Dispositif selon la revendication 1, dans lequel le premier diamètre maximal est supérieur au troisième diamètre maximal.

3. Dispositif selon la revendication 1, dans lequel un diamètre intérieur du canal augmente entre une ouverture proximale de la portion pylorique au niveau de la portion gastrique et une ouverture distale de la portion pylorique au niveau de la portion duodénale.

4. Dispositif selon la revendication 1, dans lequel le diamètre intérieur du canal diminue entre une ouverture proximale de la portion pylorique au niveau de la portion gastrique et une ouverture distale de la portion pylorique au niveau de la portion duodénale.

5. Dispositif selon la revendication 1, dans lequel l'élément de support de la portion gastrique, de la portion pylorique et de la portion duodénale comprend un seul stent de support à mémoire de forme qui s'étend d'une extrémité proximale de la portion gastrique, en passant par la portion pylorique, jusqu'à une extrémité distale de la portion duodénale.

6. Dispositif selon la revendication 1, dans lequel l'élément de support comprend trois pièces attachées de matériau de stent à mémoire de forme, une pour chacune de la portion gastrique, de la portion pylorique et de la portion duodénale.

7. Dispositif selon la revendication 1, dans lequel l'élément de support de la portion gastrique et l'élément de support de la portion duodénale comprennent chacun un matériau à mémoire de forme, et dans lequel l'élément de support de la portion pylorique comprend un matériau différent fixé à chaque extrémité à l'élément de support de la portion gastrique et à l'élément de support de la portion duodénale, dans lequel le matériau différent comprend un matériau souple, et dans lequel le matériau à mémoire de forme est choisi dans le groupe constitué du Nitinol et d'autres métaux à mémoire de forme.

8. Dispositif selon la revendication 1, dans lequel le matériau d'implant comprend deux couches de matériau, et dans lequel l'élément de support est disposé entre les deux couches.

9. Dispositif selon la revendication 1, comprenant en outre un revêtement appliqué sur au moins l'une d'une surface intérieure ou d'une surface extérieure du dispositif.

10. Dispositif selon la revendication 1, dans lequel l'élément de support est choisi dans le groupe constitué d'anneaux, d'éléments en forme d'étoile, d'un fil continu s'étendant de la portion de l'estomac à la portion duodénale et d'un fil tressé.

11. Dispositif selon la revendication 1, comprenant en outre un restricteur intégré à l'élément de support au niveau ou à proximité de la portion pylorique, dans lequel l'au moins une caractéristique du dispositif configurée pour ralentir le passage du produit alimentaire à travers le canal comprend le restricteur.

12. Dispositif selon la revendication 1, comprenant en outre un manchon duodénal (78, 1004, 1204, 1703, 1803, 1903, 2003, 2103, 2203) fixé à une extrémité distale de la portion duodénale.

13. Dispositif selon la revendication 12, comprenant en outre un restricteur (1902, 2002, 2104, 2106, 2108, 2204, 2206, 2208) couplé à ou formé dans le manchon duodénal pour ralentir le passage du produit alimentaire à travers le manchon duodénal.

14. Dispositif selon la revendication 1, dans lequel la portion pylorique est configurée pour passer d'une configuration torsadée par défaut à une configuration ouverte et non torsadée, en réponse à une quantité prédéfinie de pression appliquée à la portion gastrique par les aliments dans l'estomac, dans lequel la quantité prédéfinie de pression comprend une plage de pressions comprenant une plus petite quantité de pression à laquelle la portion pylorique commence à se détordre et une plus grande quantité de pression, qui est nécessaire pour que la portion pylorique se détorde complètement, et dans lequel la plus petite quantité de pression est comprise entre 0,0267 bar (20 mmHg) et 0,0533 bar (40 mmHg), et dans lequel la plus grande quantité de pression est d'au moins 0,1067 bar (80 mmHg).
